# EUROPEAN PATENT APPLICATION

(11) **EP 2 743 695 A1**
(43) Date of publication of application: **18.06.2014**
(21) Application number: 12382497.1
(22) Date of filing: 12.12.2012
(51) Int. Cl.: G01N 33/542, G01N 33/58

(54) **Methods and reagents for the detection of biomolecules using luminescence**

(71) Applicant: Nanogap Sub NM Powder, S.A., 15895 Ames La Coruña (ES); Pangaea Biotech S.L., 08028 Barcelona (ES)
(72) Inventor: Lopez Quintela, Manuel Arturo, 15782 Santiago de Compostela - La Coruña (ES); Domínguez Puente, Fernando, 15782 Santiago de Compostela - La Coruña (ES); Jimeno Doñaque, José Mª, 08028 Barcelona (ES)
(74) Representative: ABG Patentes, S.L.

(57) **Abstract**

The present invention relates to luminescent complexes comprising a charged transfer complex of metal atomic quantum clusters (AQCs) of at least two different sizes and a biotin-binding molecule, preferably streptavidin, and the use thereof for the detection of biotinylated compounds. The invention also relates to the use of conjugates comprising a charged transfer complex of AQCs and a biomolecule and the use thereof for the detection of binding partners of the biomolecule in a sample based on the luminescent properties of the AQCs nanosystems.

## Description

### FIELD OF THE INVENTION

The present invention relates to methods and reagents for the detection of biomolecules using binding partners for the biomolecule of interest coupled to charge-transfer complexes a metal atomic quantum clusters (AQCs). The invention also relates to competitive binding assays using charge-transfer complexes a metal atomic quantum clusters and biotinylated molecules.

### BACKGROUND OF THE INVENTION

Conjugation of fluorescent dyes to biomolecules, such as polynucleotides, proteins, lipids, etc., can be useful for detection, isolation, and/or identification of biomolecules. This conjugates generally derive from joining a dye and an agent that is capable of binding to a given target or binding partner. There are a variety of properties that might be desirable for dyes that are intended for use as markers for detection of proteins or nucleic acids. These can include ability to be excited without affecting the matrix it surrounds, easy detection, high quantum yield, adaptable to the medium, stability, long mean lifetime, non-toxicity, reproducibility of the luminescence parameters over time and the presence of a reactive group that allows attachment of the dye to a nucleotide or other desirable target

Today, the only fluorescent systems known having huge Stokes shifts of greater than 200 nm and slow decaying times of more than a microsecond are based on rare earth ions. However, they present multiple drawbacks such as: the difficulty in incorporating the same in matrices such that they do not lose their fluorescent characteristics; the existence of fixed and particular excitation, emission and Stokes shift characteristics corresponding to each rare earth, therefore they are not susceptible to being changed, and they are expensive and scarce materials. Examples of these systems are described in Sardar, D.K. et al., Biophotonics, January 2008; Resch-Genger, U., Advanced Fluorescence Reporters in Chemistry and Biology II Springer Series on Fluorescence, 2010, Volume 9, Part 1, 3-40; Harma H. et al., Analytical Chemistry, 2005, 77, 2643-2648; US7465747B2; US 2010/0224831 A1 and US 4283382.

Therefore, it would be necessary to conjugated of fluorescent dyes to biomolecules that can be used as luminescent probes and that overcome these drawbacks of the conjugates containing dyes based on rare earth elements.

### SUMMARY OF THE INVENTION

The authors of the present invention have found that, surprisingly, charge transfer complex of AQCs interact with streptavidin resulting in an increase in the intensity of their fluorescence emission and that this effect can be reverse in the presence of biotin or of a biotin binding molecule. Without wishing to be bound by any theory, it is believed that the interaction of the charge transfer complex of AQCs with streptavidin results in a conformational change in the AQCs which results in an increased intensity and that the charge transfer complexes of AQCs interact with streptavidin through the biotin-binding pockets, which results in that the interaction is reversed in the presence of biotin or of a biotin binding molecule due to the higher affinity of biotin towards streptavidin.

Since the AQCs comprise metal transition elements, these can be chosen from those which are not toxic when present in very low concentrations, such as Au or Ag. Moreover, the great natural abundance of these elements makes this a completely sustainable method. In addition, the AQCs and the complexes containing said AQCs show the following advantages:
- are stable without loss of their properties over a period of at least one year stored under natural light and room temperature,
- are stable in the pH range of 3 to 10,
- can be concentrated until dry without losing their fluorescents properties even in dried form,
- can be redissolved once dried without losing their fluorescents properties, and also
- are used at a concentration less than that used in rare earth element-based luminescent systems.

This observation allows the use of the charge transfer complex of AQCs in the presence of streptavidin or of a biotin-binding molecule for the detection of biotin or of a biotinylated molecule.

Thus, in a first aspect, the invention relates to a complex containing a biotin-binding molecule and a charge-transfer complex of at least two different size metal atomic quantum clusters (AQCs), Mₙ and M'_{n'}, of general formula (I):

Mₙ⁺M'_{n'}⁻ (I),

wherein
the metals, M and M', of the metal AQCs are the same or different metals,
Mₙ, is the smaller AQC which is present in its oxidized form, Mₙ⁺, M'_{n'}, is the larger AQC which is present in its reduced form, M'_{n'}⁻,
Mₙ⁺ and M'_{n'}⁻ are bound by electrostatic interactions,
n and n' are respectively the number of metal atoms of M and M', and
n is smaller than n'
wherein the biotin-binding molecule and the charge-transfer complex are not covalently bound.

In another aspect, the invention relates to a kit-of-parts containing, together or separately,
(i) a charge-transfer complex of at least two different size metal atomic quantum clusters (AQCs), Mₙ and M'ₙ and a biotin-binding molecule wherein the charge-transfer complex of at least two different size metal atomic quantum clusters (AQC) has the formula I as defined in claim 1 and wherein M and M', of the metal AQCs are the same or different metals, Mₙ, is the smaller AQC which is present in its oxidized form, Mₙ⁺, M'_{n'}, is the larger AQC which is present in its reduced form, M'_{n'}⁻, Mₙ⁺ and M'_{n'}⁻ are bound by electrostatic interactions, n and n' are respectively the number of metal atoms and
(ii) a biotin-binding molecule.

In another aspect, the invention relates to a method for the detection of a biotinylated molecule in a sample which comprises the steps of:
(i) contacting said sample with a complex according to the invention under conditions adequate for binding of the biotinylated molecule to the biotin-binding molecule in the complex and
(ii) detecting the change in the intensity of the fluorescence emission by the CTC following the contacting of step (i) in response to the excitation of the sample at the excitation wavelength of the CTC
wherein a decrease in the fluorescence intensity emitted by the CTC afer the contacting step is indicative of the presence in the sample of a biotinylated molecule.

In another aspect, the invention relates to a conjugate comprising a biomolecule and a charge-transfer complex of at least two different size metal AQC, Mₙ and M'_{n'}, of general formula (I):

Mₙ⁺M'_{n'}⁻ (I),

wherein
the metals, M and M', of the metal AQCs are the same or different metals, Mₙ, is the smaller AQC which is present in its oxidized form, Mₙ⁺,
M'_{n'}, is the larger AQC which is present in its reduced form, M'_{n'}⁻,
Mₙ⁺ and M'_{n'}⁻ are bound by electrostatic interactions,
n and n' are respectively the number of metal atoms of M and M', and
n is smaller than n'.
wherein the conjugate further comprises ω-hydroxyacids and ω-mercaptoacids ligands attached to the atomic quantum clusters, Mₙ and M'_{n'} and wherein the biomolecule is attached to the ω-hydroxyacids and/or to the ω-mercaptoacids ligands.

In further aspects, the invention relates to the conjugates according to the invention for detecting a biomolecule in a sample or for detecting an intracellular component.

### LEGENDS TO THE FIGURES

**Figure 1** shows the emission spectra of the AQC-CTC in the presence of streptavidin and (A) in the absence of a biotinylated oligonucleotide, (B) in the presence 0.1 mmol of biotinylated oligonucleotide and (C) in the presence of 0.5 mmol of a biotinylated oligonucleotide.
**Figure 2** shows the emission spectra of the AQC-CTC in the presence of streptavidin and HABA and (A) in the absence of a biotinylated oligonucleotide and (B) in the presence of the biotinylated oligonucleotide.

### DEFINITIONS OF TERMS

The term "antibody" as used herein refers to immunoglobulin molecules and immunologically active portions of immunoglobulin molecules, i.e., molecules that contain an antigen-binding site which specifically binds ("immunoreacts with") an antigen. Non-limiting examples include an antibodies, antibody fragments, recombinant antibodies, non-human antibodies, chimeric antibodies, humanized antibodies, or fully human antibodies. Structurally, the simplest naturally occurring antibody (e.g., IgG) comprises four polypeptide chains, two heavy (H) chains and two light (L) chains interconnected by disulfide bonds. The immunoglobulins represent a large family of molecules that include several types of molecules, such as IgD, IgG, IgA, IgM and IgE. The term "immunoglobulin molecule" includes, for example, hybrid antibodies, or altered antibodies, and fragments thereof. It has been shown that the antigen binding function of an antibody can be performed by fragments of a naturally-occurring antibody. These fragments are collectively termed "antigen-binding units". Antigen binding units can be broadly divided into "single-chain" ("Sc") and "non-single-chain" ("Nsc") types based on their molecular structures. Also encompassed within the terms "antibodies" are immunoglobulin molecules of a variety of species origins including invertebrates and vertebrates. The term "human" as applies to an antibody or an antigen binding unit refers to an immunoglobulin molecule expressed by a human gene or fragment thereof. The term "humanized" as applies to a non-human (e.g. rodent or primate) antibodies are hybrid immunoglobulins, immunoglobulin chains or fragments thereof which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat, rabbit or primate having the desired specificity, affinity and capacity. In some instances, Fv framework region (FR) residues of the human immunoglobulin are replaced by corresponding non-human residues. Furthermore, the humanized antibody may comprise residues which are found neither in the recipient antibody nor in the imported CDR or framework sequences. These modifications are made to further refine and optimize antibody performance and minimize immunogenicity when introduced into a human body. In general, the humanized antibody will comprise substantially all of at least one, and typically two, variable domains, in which all or substantially all of the CDR regions correspond to those of a non- human immunoglobulin and all or substantially all of the FR regions are those of a human immunoglobulin sequence. The humanized antibody may also comprise at least a portion of an immunoglobulin constant region (Fc), typically that of a human immunoglobulin.

As used herein, the term "approximately" means a slight variation from the specified value, preferably within 10 percent of the specified value. However, the term "approximately" may mean a greater variation tolerance depending on, for example, the experimental technique used. The person skilled in the art understands said variations of a specified value and they are within the context of the present invention. Furthermore, in order to provide a more precise description, some of the quantitative expressions provided in the present document are not described with the term "approximately". It is understood that, the term "approximately" is explicitly use or otherwise, each amount given in the present document attempts to refer to the actual given value, and it also attempts to refer to the approximation of such given value which would be reasonably deduced based on the common knowledge in the art, including equivalents and approximations due to experimental conditions and/or from measurement for such given value.

The term "Atomic Quantum Cluster", abbreviated as AQC, is understood, metal Atomic Quantum Cluster. Metal Atomic Quantum Clusters are formed exclusively by zero-oxidation-state metal atoms, in this invention preferably with equal or less than 309 metal atoms (Mₙ, n<309). The AQCs are stable over time. Preferably, the AQCs of the invention have sizes comprised between approximately 0.3 and 2.2 nm, preferably between approximately 0.3 and 2 nm, more preferably between approximately 0.3 and 1.8 nm. These metallic AQCs do not longer behave like a "metal" and their behaviour becomes molecular like. Therefore, new properties which are not observed in the nanoparticles, microparticles or metal materials in mass appear in these clusters. Therefore, the physical-chemical properties of the AQC cannot be simply extrapolated from those of the nano/microparticles.

As used herein, the term "avidin" refers to a glycoprotein found in egg white and in tissues of birds, reptiles and amphibian and which has the capacity to bind to biotin with high affinity as well as to any expressed or engineered form of the avidin biotin-binding molecule, such as streptavidin, neutravidin and the like. The term includes avidin found naturally in the eggs of *Gallus gallus* (NCBI accession numbers NM_205320.1 / GL45384353en) as well as the orthologues of said protein in other species

A "binding partner", as used herein, refers to a molecule that exhibits binding selectivity towards a binding partner or a target molecule to which it binds. A binding agent may be a biomolecule such as a polypeptide such as an antibody or protein, polypeptide-based toxin, amino acid, nucleotide, polynucleotides including DNA and RNA, lipids, and carbohydrates, or a combination thereof. A binding agent may also be a hapten, drug, ion-complexing agent such as metal chelators, microparticles, synthetic or natural polymers, cells, viruses, or other fluorescent molecules including the dye molecule according to the invention.

The term "biological sample", as used herein, refers to any sample of biological origin, including, without limitation cells in culture, bone marrow; blood; blood cells (e.g., white blood cells, red blood cells, etc.); ascites; tissue or fine needle biopsy samples; cell-containing body fluids; free floating nucleic acids; sputum; urine; cerebrospinal fluid, peritoneal fluid; pleural fluid; washings or lavages such as a ductal lavages or broncheoalveolar lavages; aspirates; scrapings; bone marrow specimens; tissue biopsy specimens; surgical specimens; other body fluids, secretions, and/or excretions; and/or cells therefrom. In some embodiments, a sample comprises cells obtained from a patient. The cells may be, for example, from blood, bone marrow, and/or from tissue derived from solid organs, such as brain, spleen, bone, heart, vascular, lung, kidney, liver, pituitary, endocrine glands, lymph node, dispersed primary cells, tumor cells. Biological samples may include sections of tissues, including but not limited to frozen or fixed sections taken for histological purposes. In some embodiments, a sample may be a body fluid, including, but not limited to, blood fluids, lymph, ascitic fluids, gynecological fluids, and urine. Samples may be obtained from a subject by any of a wide variety of methods known in the art, including without limitation biopsy (e.g., fine needle aspiration or tissue biopsy), surgery, and collection of body fluid (e.g., blood, lymph, etc.). Biological samples also include any material derived by processing any of the above samples. Derived samples may, for example, include nucleic acids or proteins extracted from the biological sample, or obtained by subjecting the sample to techniques such as amplification or reverse transcription of mRNA, or isolation and/or purification of certain components.

The term "biomolecule" is used herein as known to the expert skilled in the art and refers to any organic molecule that is produced by a living organism or to any artificially produced derivatives of such compounds, including large polymeric molecules such as proteins, polysaccharides, carbohydrates, lipids, nucleic acids and oligonucleotides as well as small molecules such as primary metabolites, secondary metabolites, and natural products.

The term "biotin-binding molecule" as used herein is intended to encompass any compound which is capable of tightly but non-covalently binding to biotin or any biotin compound.

The term "biotinylated molecule" is to be understood as a conjugate of modified biotin or a biotin analog with another moiety such as biomolecules, e. g. nucleic acid molecules (including single or double stranded DNA, RNA, DNA/RNA chimeric molecules, nucleic acid analogs and any molecule which contains or incorporates a nucleotide sequence, e. g. a peptide nucleic acid (PNA) or any modification thereof), proteins (including glycoproteins, enzymes, peptides library or display products and antibodies or derivatives thereof), peptides, carbohydrates or polysaccharides, lipids, etc., wherein the other moieties are covalently linked to the modified biotin or biotin analogues. Many biotinylated ligands are commercially available or can be prepared by standard methods. Processes for coupling a biomolecule, e. g. a nucleic acid molecule or a protein molecule, to biotin are well known in the art (Bayer and Wilchek, Methods in Molec. Biology 10, 143. 1992).

The term "charge-transfer complex" also named CT complex, or electron-donor-acceptor complex is herein understood as an association of at least two AQCs, in which a fraction of electronic charge, i.e. an electron, is transferred between the AQCs resulting in the formation of the oxidized form of one of the AQCs and the reduced form of the other AQC. The resulting electrostatic interaction, i.e. electrostatic attraction, provides a stabilizing force for the molecular complex. The source AQC from which the charge is transferred is called the electron donor and the receiving AQC is called the electron acceptor. In the present invention:
- Mₙ is the electron donor, which is the smaller AQC within the complex, and
- M'_{n'} is the electron acceptor, which is the larger AQC within the complex.

The terms "detect", "detecting" and the like encompass quantitative as well as qualitative measurements.

As used herein "fluorescent in situ hybridization" or "FISH" refers to a method for detecting or localizing a specific DNA sequence on a chromosome through the use of a labeled nucleic acid probe that hybridizes to a specific DNA sequence on a chromosome.

By "immobilized" is meant bound directly or indirectly to a surface of, e.g., a device, including attachment by covalent binding or noncovalent binding (e.g., hydrogen bonding, ionic interactions, van der Waals forces, or hydrophobic interactions). By "posttranslational modification" is meant chemical modification of a protein after its translation. This includes, but is not limited to, the addition of functional groups (e.g., phosphorylation, glycosylation, acetylation, alkylation, methylation, formylation, oxidation, or biotinylation), addition of proteins or peptides (e.g., ubiquitination), changing the chemical nature of amino acids (e.g., deamination or demethylation), and structural changes (e.g., disulfide bridges or proteolytic cleavage).

As used herein the term "immunohistochemistry (IHC)" also known as "immunocytochemistry (ICC)" when applied to cells refers to a tool in diagnostic pathology, wherein panels of monoclonal antibodies can be used in the differential diagnosis of undifferentiated neoplasms (e.g., to distinguish lymphomas, carcinomas, and sarcomas) to reveal markers specific for certain tumor types and other diseases, to diagnose and phenotype malignant lymphomas and to demonstrate the presence of viral antigens, oncoproteins, hormone receptors, and proliferation-associated nuclear proteins.

The term "kit", as used herein, refers to a product containing the different reagents necessary for carrying out the methods of the invention packed so as to allow their transport and storage. Materials suitable for packing the components of the kit include crystal, plastic (e.g. polyethylene, polypropylene, polycarbonate), bottles, vials, paper, or envelopes.

As used herein a "nucleic acid probe" refers to a nucleic acid (such as DNA, RNA, PNA etc.) sequence that recognizes and hybridizes to a specific DNA sequence on a chromosome.

The term "spacing group" or "spacer" refers to a portion of a chemical structure which connects two or more substructures. These spacer groups will be enumerated hereinafter in this application. The atoms of a spacing group and the atoms of a chain within the spacing group are themselves connected by chemical bonds. Among the preferred spacers are straight or branched, saturated or unsaturated, carbon chains. Theses carbon chains may also include one or more heteroatoms within the chain or at termini of the chains. By "heteroatoms" is meant atoms other than carbon which are chosen from the group consisting of oxygen, nitrogen and sulfur. Spacing groups may also include cyclic or aromatic groups as part of the chain or as a substitution on one of the atoms in the chain.

The term "polynucleotide" as used herein are used interchangeably refers to deoxyribonucleic acid in its various forms as understood in the art, such as genomic DNA, cDNA, isolated nucleic acid molecules, vector DNA, and chromosomal DNA. "Nucleic acid" refers to DNA or RNA in any form. Examples of isolated nucleic acid molecules include, but are not limited to, recombinant DNA molecules contained in a vector, recombinant DNA molecules maintained in a heterologous host cell, partially or substantially purified nucleic acid molecules, and synthetic DNA molecules. Typically, an "isolated" nucleic acid is free of sequences which naturally flank the nucleic acid (i.e., sequences located at the 5' and 3' ends of the nucleic acid) in the genomic DNA of the organism from which the nucleic acid is derived. Moreover, an "isolated" nucleic acid molecule, such as a cDNA molecule, is generally substantially free of other cellular material or culture medium when produced by recombinant techniques, or free of chemical precursors or other chemicals when chemically synthesized.

The terms "polypeptide", "peptide" and "protein" are used interchangeably herein to refer to polymers of amino acids of any length. The polymer may be linear, cyclic, or branched. The polymer may comprise modified amino acids, and it may be interrupted by non-amino acids. The terms also encompass amino acid polymers that have been modified, for example, via sulfonation, glycosylation, lipidation, acetylation, phosphorylation, iodination, methylation, oxidation, proteolytic processing, phosphorylation, prenylation, racemization, selenoylation, transfer-RNA mediated addition of amino acids to proteins such as arginylation, ubiquitination, or any other manipulation, such as conjugation with a labeling component. As used herein the term "amino acid" refers to either natural and/or unnatural or synthetic amino acids, including glycine and both the D or L optical isomers, and amino acid analogs and peptidomimetics.

The term "protecting group", as used heren, refers to a grouping of atoms that when attached to a reactive group in a molecule masks, reduces or prevents that reactivity.

The term "reactive group" as used herein refers to a group that is capable of reacting with another chemical group to form a covalent bond, i.e. is covalently reactive under suitable reaction conditions, and generally represents a point of attachment for another substance. Reactive groups generally include nucleophiles, electrophiles and photoactivatable groups. Exemplary reactive groups include, but not limited to, olefins, acetylenes, alcohols, phenols, ethers, oxides, halides, aldehydes, ketones, carboxylic acids, esters, amides, cyanates, isocyanates, thiocyanates, isothiocyanates, amines, hydrazines, hydrazones, hydrazides, diazo, diazonium, nitre, nitriles, mercaptans, sulfides, disulfides, sulfoxides, sulfones, sulfonic acids, sulfuric acids, acetals, ketals, anhydrides, sulfates, sulfenic acids isonitriles, amidines, imides, imidates, nitrones, hydroxylamines, oximes, hydroxamic acids thiohydroxamic acids, allenes, ortho esters, sulfites, enamines, ynamines, ureas, pseudoureas, semicarbazides, carbodiimides, carbamates, imines, azides, azo compounds, azoxy compounds, and nitroso compounds. Reactive functional groups also include those used to prepare bioconjugates, e.g., N-hydroxysuccinimide esters, maleimides and the like. Methods to prepare each of these functional groups are well known in the art and their application to or modification for a particular purpose is within the ability of one of skill in the art (see, for example, Sandler and Karo, eds., Organic Functional Group Preparations. Academic Press, San Diego, 1989).

The term "sample", as used herein, is used in its broadest sense and refers to a sample containing a biomolecule which is to be detected. In some embodiments, the sample is an environmental sample such as soil, water, or air; or from an industrial source such as taken from a waste stream, a water source, a supply line, or a production lot. In another embodiment, the sample is a biological sample.

As used in the present invention, the expression "specific binding" refers to the capacity of a first molecule to bind specifically to a second molecule by means of the existence of complementarity between the three-dimensional structures of the two molecules with a substantially higher affinity than for non-specific binding such that the binding between said first and second molecule preferably takes place before the binding of any of said molecules with respect to the other molecules present in the reaction mixture. It is understood that there is high affinity in the binding of two molecules when the complex resulting from said binding has a dissociation constant (KD) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M or less than 10⁻¹⁵ M.

The term "streptavidin", as used herein, corresponds to the protein from *Streptomyces avidinii* (accession number CAA00084.1 in GenBank), as well as the orthologues, homologues and fragments of streptavidin defined in the same manner as avidin.

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C., with washes at 42°C in 0.2xSSC (sodium chloride/sodium citrate) and 50% formamide, followed by a high-stringency wash consisting of 0.1xSSC containing EDTA at 55 °C. Moderately stringent conditions may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C. in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, etc. as necessary to accommodate factors such as probe length and the like. In some embodiments, the nucleotide which is used as binding partner for the detection of the biomolecule may be of any length, such as at least 5, a least 6, at least 7, at least 8, at least 9, at least 10, at least 12, at least 15, at least 20, at least 25, at least 30, at least 35, at least 40, at least 50, at least 60, at least 70, at least 80, at least 90, at least 100, at least 120, at least 150, at least 200, etc., nucleotides in length. The nucleic acid may comprise deoxyribonucleic acid (DNA), ribonucleic acid (RNA), peptide nucleic acid (PNA), derivatives of any of the foregoing, and combinations of any of the foregoing. Regardless of the composition of the oligonucleotide, each unit of the oligonucleotide that is equivalent to a DNA or RNA base is referred to as a "nucleotide."

The term "tag", as used herein, relates to any amino acid sequence for which specific binding molecules are available, thus allowing the detection/purification of any polypeptide carrying said tag.

The term "ω-hydroxyacid", as used herein, refers to a compound having the general formula HO-(CH₂)ₘ-COOH where m has a value between 2 and 30.

The term "ω-mercaptoacids", as used herein, refers to a compound having the general formula HS-(CH₂)ₚ-COOH where m hase a value between 2 and 30.

### DETAILED DESCRIPTION OF THE INVENTION

### Non-covalent complexes of AQC-CTCs and biotin-binding molecules

The authors of the present invention have observed that streptavidin interacts with AQC-CTCs leading to an increase in their fluorescence intensity and that this binding can be reversed in the presence of biotin or biotinylated molecules, thereby resulting in a decrease in the fluorescence intensity of the complexes comprising AQC-CTCs and streptavidin.

Thus, in a first aspect, the invention relates to a complex containing a biotin-binding molecule and a charge-transfer complex of at least two different size metal atomic quantum clusters (AQCs), Mₙ and M'_{n'}, of general formula (I):

Mₙ⁺M'_{n'}⁻ (I),

wherein
the metals, M and M', of the metal AQCs are the same or different metals,
Mₙ, is the smaller AQC which is present in its oxidized form, Mₙ⁺,
M'_{n'}, is the larger AQC which is present in its reduced form, M'_{n'}⁻,
Mₙ⁺ and M'_{n'}⁻ are bound by electrostatic interactions,
n and n' are respectively the number of metal atoms of M and M', and
n is smaller than n'
wherein the biotin-binding molecule and the charge-transfer complex are not covalently bound.

In one embodiment the metals, M and M', of the metallic AQCs are selected from transition metals or combinations thereof, preferably the transition metals are selected from the group consisting of Au, Ag, Co, Cu, Pt, Fe, Cr, Pd, Ni, Rh and combinations thereof, more preferably they are selected from the group consisting of Au, Ag, Cu and combinations thereof, and more preferably the transition metals are selected from the group consisting of Au, Ag and the combination thereof.

Since the excitation and emission wavelengths of the fluorescence depend on the size of the AQCs, it is possible to select the excitation and emission wavelengths by directing the formation of AQCs of necessary sizes.

In a preferred embodiment, only one electron is transferred between the at least two AQCs, Mₙ and M'_{n'}, therefore resulting the ionic forms, Mₙ⁺, i.e. the oxidized form of Mₙ, and M'_{n'}⁻, the reduced form of M'_{n'}, wherein "+" is a positive charge and "-" is a negative charge.

In some embodiments, n and n' are between 2 and 309, between 2 and 102, between 2 and 55, or between 2 and 25 metal atoms. In some embodiments, the difference between n and n' is between 5 and 50 atoms.

In a further embodiment the difference between n and n' is between 5 and 50 atoms or between 5 and 25 atoms.

In some embodiments, the CTCs further comprise ω-hydroxyacids and ω-mercaptoacids ligands attached to the atomic quantum clusters, Mₙ and M'_{n'}.

In some embodiments, the biotin-binding molecule is avidin, streptavidin or any variant thereof which substantially preserves the biotin-binding capacity and which interacts with AQC-CTCs in a manner that leads to a change in at least one of the parameters of the fluorescence emission of the AQC-CTCs. Methods for detecting whether an avidin or streptavidin analog or variant preserve their biotin-binding capacity are widely known in the art and include, for instance, the method described in W02012058635 based in BioLayer Interferometry (BLI), wherein a layer of molecules bound to the tip of an optic fiber creates an interference pattern in white light reflected from the layer of molecules. A change in the number of molecules bound to the tip of the optic fiber causes a shift in that interference pattern that can be measured. The wavelength shift is a direct measure of the thickness of the layer of molecules. Further, because the shift can be measured in real-time, rates of association and dissociation can be determined without further ado.

Methods suitable for determining whether a given avidin or streptavidin variant which interacts with AQC-CTCs in a manner that leads to a change in at least one of the parameters of the fluorescence emission of the AQC-CTCs are also available to the skilled person and include, for instance, the method described in example 1 of the present application, wherein the AQC-CTCs is contacted with the streptavidin variant and the change (increase) in fluorescence intensity is determined.

In some embodiments, the biotin-binding molecule is a streptavidin or an avidin fragment which retains substantial binding activity for biotin, such as at least 50 percent or more of the binding affinity of native streptavidin or avidin, respectively, may also be used. Preferably, the affinity of the avidin variant for biotin is of at least 10¹⁵ M⁻¹, 10¹⁴ M⁻¹, 10¹³ M⁻¹, 10¹² M⁻¹, 10¹⁰ M⁻¹ or 10⁹ M⁻¹.

For convenience, in the instant description, the terms "avidin" and "streptavidin" as used herein are intended to encompass biotin-binding fragments, mutants and core forms of these binding pair members wherein the biotin-binding capacity and the effect on the fluorescence emission by the AQC-CTCs is substantially preserved. Avidin and streptavidin are available from commercial suppliers. Moreover, the nucleic acid sequences encoding streptavidin and avidin and the streptavidin and avidin amino acid sequences can be found, for example, in GenBank Accession Nos. X65082; X03591; NM --205320; X05343; Z21611; and Z21554.

In some embodiments, avidin and streptavidin variants suitable for use in the present invention include, without limitation
- "Core streptavidin", which is a truncated version of the full-length streptavidin polypeptide which may include streptavidin residues 13-138, 14-138, 13-139 and 14-139. See, e.g., Pahler et al., (JBiol Chem 1987:262:13933-37).
- Truncated forms of streptavidin and avidin that retain strong binding to biotin (See, e.g. Sano et al., (J Biol Chem 1995;270:28204-09) (describing core streptavidin variants 16-133 and 14-138) (U.S. Pat. No. 6,022,951).
- Mutants of streptavidin and core forms of streptavidin which retain substantial biotin binding activity or increased biotin binding activity. See Chilcoti et al., Proc Natl Acad Sci USA 1995;92(5):1754-8; Reznik et al., Nat Biotechnol 1996;14(8):1007-1011*.*
- Mutants with reduced immunogenicity, such as mutants modified by site-directed mutagenesis to remove potential T cell epitopes or lymphocyte epitopes. See Meyer et al., Protein Sci 2001;10:491-503.
- Mutants of avidin and core forms of avidin which retain substantial biotin binding activity or increased biotin binding activity also may be used. See Hiller et al., JBiochem 1991;278:573-85; Livnah et al. Proc Natl Acad Sci USA 1993;90:5076-80.
- Variants resulting from the chemical modification of avidin such as those resulting from the complete or partial modification of glycosylation and fragments thereof as well as the completely deglycosylated avidin variant known as neutravidin.
- Avidin mutants as described in WO05047317A1
- Avidin-like proteins as described in W006045891,
- Recombinant avidin as described in W00198349,
- Avidin variants as described in W00027814,
- Monomeric streptavidin as described in W006084388,
- Modified streptavidin dimers such as those described in W006058226,
- The protein with biotin binding capacity as described in W004018509,
- Streptavidin having a higher affinity for biotin as described in W09840396,
- The modified streptavidin and avidin molecules as described in W09640761,
- The streptavidin mutants as described in W09711183,
- The streptavidin with modified affinity as described in W09624606.

Different avidin variants are commercially available, such as Extravidin (Sigma-Aldrich), NeutrAvidin (Thermo Scientific), NeutrAvidin (Invitrogen) and NeutraLite (Belovo).

In some embodiments, the biotin-binding molecule is a functionally equivalent variant of streptavidin. As it is used herein, "functionally equivalent variant of streptavidin is understood as a polypeptide which substantially preserves the biotin binding capacity of streptavidin. The functionally equivalent variants of streptavdinV include polypeptides showing at least 60%, 65%, 70%, 72%, 74%, 76%, 78%, 80%, 90%, 95%, 97%, 99% similarity or identity with strpetavidin over their complete length.

The term "not-covalently bound", as used herein is used to indicate that the biotin-binding molecule and the charge-transfer complex interact by intermolecular forces which do not involve a covalent bond between an atom of the CTC-AQC and an atom of the biotin binding molecule. In some embodiments, the CTC-AQC and the biotin binding molecule are connected by hydrogen bonds, by hydrophobic interactions, by ionic bond or a combination thereof.

In some embodiments, the CTC further comprise organic ligands attached thereto. The "organic ligands" that may be attached to the CTC are at least two different types of organic ligands, and preferably the at least two different types of organic ligands are selected from ω-hydroxyacid (HO-(CH₂)ₘ-COOH) and ω-mercaptoacids (HS-(CH₂)ₚ-COOH) ligands where m and p have a value between 2 and 30, preferably m and p have a value between 10 and 20. In a particular embodiment m and p have a value of 15. In another particular embodiment m and p have a value of 11. The value of m and p can be different or the same. In the event that m and p are different the difference between them is less than 6 carbons, preferably the difference of the values of m and p is between 1 and 4. In a preferred embodiment m and p are the same. Wherein the at least two different types of organic ligands are selected from ω-hydroxyacid (HO-(CH₂)ₘ-COOH) and ω-mercaptoacid (HS-(CH₂)ₚ-COOH) ligands, the acid groups, -COOH, (or -COO⁻, if the salt of the corresponding acid is used) are directed towards the outer surface of the nanocompound and the -OH and -SH groups directed towards the inside, i.e. towards the ionized AQCs, Mₙ⁺ and M'_{n'}⁻, being bound, attached or coordinated to them.

In another embodiment the "organic ligands" that may be attached to the charge-transfer complex have other functional groups than hydroxyl, -OH, or mercapto, -SH groups, such as -NH₂, -NH-, -Cl, -PH₃, -SR, -OR, -NR₂, -NHR, -NR-, wherein R represents an organic group of a short hydrocarbon chain, C₁-C₄ capable of bound, attach or coordinate the AQCs or the ionized AQCs, Mₙ⁺ and M'_{n'}⁻. Is also possible exchanging the hydroxyl, -OH, or mercapto, -SH groups of the ω-hydroxyacid (HO-(CH₂)ₘ-COOH) and ω-mercaptoacid (HS-(CH₂)ₚ-COOH) ligands with these others, mentioned above that also interact with the metals of the AQCs.

In some embodiments, when the biotin-binding molecule is streptavidin, the complex according to the invention is a homotetramer containing 4 streptavidin monomers and having a variable number of CTC-ACQs. In some embodiments, the complex is formed by a homotetramer of streptavidin and at least 1, at least 2, at least 3, at least 4, at least 8, at least 12, at least 16, at least 20 or more CTC-ACQs per complex.

### Kit-of-parts comprising AQC-CTCs and biotin-binding molecule

In another embodiment, the invention relates to a kit-of-parts comprising, together or separately,
(i) a charge-transfer complex of at least two different size metal atomic quantum clusters (AQCs), Mₙ and M'ₙ and a biotin-binding molecule wherein the charge-transfer complex of the at least two different size metal atomic quantum clusters (AQC) has the formula I as defined above and wherein M and M', of the metal AQCs are the same or different metals, Mₙ, is the smaller AQC which is present in its oxidized form, Mₙ⁺, M'_{n'}, is the larger AQC which is present in its reduced form, M'_{n'}⁻, Mₙ⁺ and M'_{n'}⁻ are bound by electrostatic interactions, n and n' are respectively the number of metal and
(ii) a biotin-binding molecule.

In some embodiments, the biotin-binding molecule is streptavidin or any of the biotin-binding molecules which are referred to in the context of the complex of the invention.

In some embodiments, the metals, M and M', of the metal atomic quantum clusters are independently selected from transition metals or combinations thereof. In some embodiments, the metals M and M', of the metal atomic quantum clusters are independently selected from the transition metals Au, Ag, Cu and combinations thereof, and more preferably Au, Ag and the combination thereof. In some embodiments, n and n' take values between 2 and 309, between 2 and 102, between 2 and 55, or between 2 and 25 metal atoms. In some embodiments, the difference between n and n' is between 5 and 50 atoms. In some embodiments, the charge-transfer complex further comprise ω-hydroxyacids andr ω-mercaptoacids ligands attached to the atomic quantum clusters, Mₙ and M'_{n'} having the general formula HO-(CH₂)ₘ-COOH where m has a value between 2 and 30 or HS-(CH₂)ₚ-COOH where m hase a value between 2 and 3, wherein the different embodiments, concering the values of m and p and the difference between said values is as described in the context of the complex of the invention.

In some embodiments, the components of the kit are found in separate containers.

Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

### Competitive assays for the detection of biotinylated molecules using AQC-CTCs

The authors of the present invention have observed that the fluorescence intensity of AQC-CTCs is increased in the presence of streptavidin and that this increase in the fluorescence is reversed in the presence of biotin or of a biotinylated molecule, thereby allowing the detection of biotin or of biotinylated molecules in a sample by measuring the change in fluorescence intensity of the AQC-CTCs. Without wishing to be bound by any theory, it is thought that the AQC-CTCs are capable of interacting with the biotin-binding molecule in a way that results in an enhancement of the fluorescence intensity emitted by the AQC-CTCs. This binding is reversed in the presence of biotin or a biotin analogue, which leads to the release of the AQC-CTCs and to a decrease in their fluorescence.

In an aspect, the invention relates to a method for the detection of a biotinylated molecule in a sample which comprises the steps of:
(i) contacting said sample with a complex according to any of claims 1 to 4 under conditions adequate for binding of the biotinylated molecule to the biotin-binding molecule in the complex and
(ii) detecting the change in the intensity of the fluorescence emission by the ACQ following the contacting of step (i) in response to the excitation of the sample at the excitation wavelength of the ACQ
wherein a decrease in the fluorescence intensity emitted by the ACQ after the contacting step is indicative of the presence in the sample of a biotinylated molecule.

In a first step, the sample suspected of containing a biotinylated molecule is contacted with a composition comprising a biotin-binding molecule and CTC-AQCs.

In some embodiments, the metals M and M' of the metal atomic quantum clusters are independently selected from transition metals or combinations thereof. In some embodiments, M and M' are independently selected from the transition metals Au, Ag, Cu and combinations thereof, and more preferably Au, Ag and the combination thereof.

In some embodiments, n and n' are between 2 and 309, between 2 and 102, between 2 and 55, or between 2 and 25 metal atoms. In some embodiments, the difference between n and n' is between 5 and 50 atoms.

In some embodiments, the AQC-CTCs further comprise ω-hydroxyacids and ω-mercaptoacids ligands attached to the atomic quantum clusters, Mₙ and M'_{n'}. In some embodiments, the ω-hydroxyacids andr ω-mercaptoacids ligands attached to the atomic quantum clusters, Mₙ and M'_{n'} have the general formula HO-(CH₂)ₘ-COOH where m has a value between 2 and 30 or HS-(CH₂)ₚ-COOH where m hase a value between 2 and 3, wherein the different embodiments, concering the values of m and p and the difference between said values is as described in the context of the complex of the invention.

In some embodiments, the biotin-binding molecule is avidin, streptavidin or any variant thereof which substantially preserves the biotin-binding capacity and which interacts with AQC-CTCs in a manner that leads to a change in at least one of the parameters of the fluorescence emission of the AQC-CTCs. Methods for detecting whether an avidin or streptavidin analog presever their biotin-binding capacity are widely known in the art and include, for instance, the method described in W02012058635 based in BioLayer Interferometry (BLI), wherein a layer of molecules bound to the tip of an optic fiber creates an interference pattern in white light reflected from the layer of molecules. A change in the number of molecules bound to the tip of the optic fiber causes a shift in that interference pattern that can be measured. The wavelength shift is a direct measure of the thickness of the layer of molecules. Further, because the shift can be measured in real-time, rates of association and dissociation can be determined

Methods suitable for determining whether a given avidin or streptavidin variant which interacts with AQC-CTCs in a manner that leads to a change in at least one of the parameters of the fluorescence emission of the AQC-CTCs are also available to the skilled person and include, for instance, the method described in example 1 of the present application, wherein the AQC-CTCs is contacted with the streptavidin variant and the change (increase) in fluorescence intensity is determined.

In some embodiments, the biotin-binding molecule is a streptavidin or an avidin fragment which retains substantial binding activity for biotin, such as at least 50 percent or more of the binding affinity of native streptavidin or avidin, respectively, may also be used. Preferably, the affinity of the avidin variant for biotin is of at least 10¹⁵ M⁻¹, 10¹⁴ M⁻¹, 10¹³ M⁻¹, 10¹² M⁻¹, 10¹⁰ M⁻¹ or 10⁹ M⁻¹.

In some embodiments, the "biotin-binding molecule" is as defined in the context of the non-covalent complexes of AQC-CTCs and biotin-binding molecule. In some embodiments, avidin and streptavidin variants suitable for use in the present invention include, without limitation, Core streptavidin, truncated forms of streptavidin and avidin (See, e.g. Sano et al., (J Biol Chem 1995;270:28204-09) (describing core streptavidin variants 16-133 and 14-138) (U.S. Pat. No. 6,022,951), mutants of streptavidin and core forms of streptavidin which retain substantial biotin binding activity or increased biotin binding activity. See Chilcoti et al., Proc Natl Acad Sci USA 1995;92(5):1754-8; Reznik et al., Nat Biotechnol 1996;14(8):1007-1011, mutants of streptavidin and core forms of streptavidin with reduced immunogenicity, such as mutants modified by site-directed mutagenesis to remove potential T cell epitopes or lymphocyte epitopes. See Meyer et al., Protein Sci 2001;10:491-503; mutants of avidin and core forms of avidin which retain substantial biotin binding activity or increased biotin binding activity also may be used. See Hiller et al., J Biochem 1991;278:573-85; Livnah et al. Proc Natl Acad Sci USA 1993;90:5076-80, variants resulting from the chemical modification of avidin such as those resulting from the complete or partial modification of glycosylation and fragments thereof as well as the completely deglycosylated avidin variant known as neutravidin, avidin mutants as described in W005047317A1, avidin-like proteins as described in W006045891, recombinant avidin as described in W0200198349, avidin variants as described in W00027814, monomeric streptavidin as described in W006084388, modified streptavidin dimers such as those described in W006058226, the biotin-biding protein as described in W004018509, streptavidin having a higher affinity for biotin as described in W09840396, the modified streptavidin and avidin molecules as described in W09640761, the streptavidin mutants as described in W09711183, the streptavidin with modified affinity as described in W09624606 as well as different avidin variants are commercially available, such as Extravidin (Sigma-Aldrich), NeutrAvidin (Thermo Scientific), NeutrAvidin (Invitrogen) and NeutraLite (Belovo).

In some embodiments, the biotinylated molecule is an an amino acid, a peptide, a protein, a tyramine, a polysaccharide, an ion-complexing moiety, a nucleoside, a nucleotide, an oligonucleotide, a nucleic acid, a hapten, a psoralen, a drug, a hormone, a lipid, a lipid assembly, a polymer, a polymeric microparticle, a biological cell or virus. More typically, substrate is a peptide, a protein, a nucleotide, an oligonucleotide, or a nucleic acid.

In one embodiment, the biotinylated molecule is an amino acid (including those that are protected or are substituted by phosphonates, carbohydrates, or C1 to C25 carboxylic acids), or is a polymer of amino acids such as a peptide or protein. Preferred conjugates ofpeptides contain at least five amino acids, more preferably 5 to 36 amino acids. Preferred peptides include, but are not limited to, neuropeptides, cytokines, toxins, protease substrates, and protein kinase substrates. Preferred protein conjugates include enzymes, antibodies, lectins, glycoproteins, histones, albumins, lipoproteins, avidin, streptavidin, protein A, protein G, phycobiliproteins and other fluorescent proteins, hormones, toxins, chemokines and growth factors. In one preferred aspect, the conjugated protein is a phycobiliprotein, such as allophycocyanin, phycocyanin, phycoerythrin, allophycocyanin B, B-phycoerythrin, and phycoerythrocyanin, (for example, see U.S. Pat. No. 5,714,386 to Roederer (1998)).

In one embodiment, the biomolecule is an antibody (including intact antibodies, antibody fragments, and antibody sera, etc.), an amino acid, an angiostatin or endostatin, an avidin or streptavidin, a biotin (e.g. an amidobiotin, a biocytin, a desthiobiotin, etc.), a blood component protein (e.g. an albumin, a fibrinogen, a plasminogen, etc.), a dextran, an enzyme, an enzyme inhibitor, an IgG-binding protein (e.g. a protein A, protein G, protein A/G, etc.), a fluorescent protein (e.g. a phycobiliprotein, an aequorin, a green fluorescent protein, etc.), a growth factor, a hormone, a lectin (e.g. a wheat germ agglutinin, a conconavalin A, etc.), a lipopolysaccharide, a metal-binding protein (e.g. a calmodulin, etc.), a microorganism or portion thereof (e.g. a bacteria, a virus, a yeast, etc.), a neuropeptide and other biologically active factors (e.g. a dermorphin, a deltropin, an endomorphin, an endorphin, a tumor necrosis factor etc.), a non-biological microparticle (e.g. of ferrofluid, gold, polystyrene, etc.), a nucleotide, an oligonucleotide, a peptide toxin (e.g. an apamin, a bungarotoxin, a phalloidin, etc.), a phospholipid-binding protein (e.g. an annexin, etc.), a small-molecule drug (e.g. a methotrexate, etc.), a structural protein (e.g. an actin, a fibronectin, a laminin, a microtubule-associated protein, a tublin, etc.), or a tyramide.

In another embodiment, the biomolecule is a nucleic acid base, nucleoside, nucleotide or a nucleic acid polymer. Preferred nucleic acid polymers are single- or multi-stranded, natural or synthetic DNA or RNA, DNA or RNA oligonucleotides, or DNA/RNA hybrids, or incorporate an unusual linker such as morpholine derivatized phosphates, or peptide nucleic acids such as N-(2-aminoethyl)glycine units. When the nucleic acid is a synthetic oligonucleotide, it typically contains fewer than 50 nucleotides, more typically fewer than 25 nucleotides.

In another embodiment, the biomolecule is a carbohydrate that is typically a polysaccharide, such as a dextran, heparin, glycogen, amylopectin, mannan, inulin, starch, agarose and cellulose. Alternatively, the carbohydrate is a polysaccharide that is a lipopolysaccharide. Preferred polysaccharide conjugates are dextran, or lipopolysaccharide conjugates.

In some embodiments, the biotin-binding molecule is provided as a complex with a biotin-analogue showing an affinity towards the biotin-binding molecule which is lower than that of biotin. These biotin analogues are capable of binding to the biotin-binding molecule in the absence of biotin. If the sample contains a biotinylated molecule, biotin displaces the analogue from the biotin-binding molecule due to its higher affinity for the biotin-binding sites, which results in an decrease in the emission maximum intensity, due to the interaction between the oligo-biotin and the streptavidin

Suitable biotin analogues include compounds which bind the biotin-binding molecule with a K_{D} of greater than 1 x 10⁻¹³ M. In some embodiments, the biotin analog binds the biotin-binding molecule with a K_{D} of between 1 x 10⁻¹³ M and 1 x 10⁻⁸ M, between 1 x 10⁻¹² M and 1 x 10⁻⁹ M, or between 1 x 10⁻¹¹ M and 1 x 10⁻⁹ M, between 10⁻¹³ M to 10⁻⁴ M, between 10⁻¹² M to 10⁻⁴, between 10⁻¹¹ M to 10⁻⁴ M, between 10⁻¹⁰ M to 10⁻⁴ M, between 10⁻⁹ M to 10⁻⁴ M, between 10⁻⁸ M to 10⁻⁴ M, between 10⁻⁷ M to 10⁻⁴ M, or between 10⁻⁶ M to 10⁻⁴ M. Suitable biotin analogues for use in the present invention include, without limitation, HABA (4-hydroxyazobenzene-2-carboxylic acid), 2-azidobiotin, 2-azidobiotinyl adenylate, 2-propargyl (2-propynyl) biotin, strep-tag II peptide sequence (Trp-Ser-His-Pro-Glu-Phe-Glu-Lys) (SEQ ID NO:1) (Schmidt TGM and Skerra A, Nature Prot. 2007, 2, 1528-1535), 2-iminobiotin, biocytin (- biotinoyl-L-lysine), biotin ethylenediamine, biotin cadaverine, biotin-X cadaverine, DSB-X desthiobiocytin (-desthiobiotinoyl-L-lysine,) and DSB-X biotin ethylenediamine and chloroacetylated biotin derivative (CABI) or the biotin analogues described in W02012058635.

In some embodiments, the biotin-binding molecule is bound to a solid support. Non-limiting exemplary solid supports include polymers (such as agarose, sepharose, cellulose, nitrocellulose, alginate, Teflon, latex, acrylamide, nylon, plastic, polystyrene, silicone, etc.), glass, silica, ceramics, and metals. Such solid supports may take any form, such as particles (including microparticles), sheets, dip-sticks, gels, filters, membranes, microfiber strips, tubes, wells, plates (such as microplates, including 6-well plates, 24-well plates, 96-well plates, 384-well plates, etc.), fibers, capillaries, combs, pipette tips, microarray chips, etc. In some embodiments, the biotin-binding moiety is associated with the surface of a solid support. In some embodiments, the surface of the solid support comprises an irregular surface, such as a porous, particulate, fibrous, webbed, or sintered surface.

In some embodiments, a solid support is selected from a microplate, a microarray chip, and a microparticle. In some embodiments, a solid support is at least partially composed of a polymer. In some embodiments, a microparticle solid support comprises monodisperse or polydisperse spherical beads. Monodisperse microparticles are substantially uniform in size (i.e., they have a diameter standard deviation of less than 5 percent), while polydisperse microparticles vary in size. In some embodiments, microparticles are composed of the same polymer throughout, or are core-shell polymers, in which the core of the microparticle is composed of one polymer, and the outer layer (or "shell") is composed of another. In some embodiments, microparticles are magnetic.

In some embodiments, a biotin-binding moiety is attached to a solid support through an amino or sulfhydryl group of the biotin-binding moiety. In some such embodiments, the surface of the solid support comprises a group capable of reacting with a free amine or sulfhydryl group. Nonlimiting exemplary such groups include carboxy, active halogen, activated 2-substituted ethylsulfonyl, activated 2-substituted ethyl carbonyl, active ester, vinylsulfonyl, vinylcarbonyl, aldehyde, epoxy, etc. Some such groups may require the use of an additional reactant to render the group capable of reacting with a free amine or sulfhydryl group. Nonlimiting exemplary additional reactants include cyanogen bromide, carbonyldiimidazole, glutaraldehyde, hydroxylsuccinimide, tosyl chloride, etc.

Many solid supports are known in the art, and one skilled in the art can select a suitable solid support according to the intended application. Similarly, if the solid support is not commercially available with a biotin-binding moiety attached to its surface, one skilled in the art can select a suitable method of attaching a biotin-binding moiety to a solid surface. Exemplary such methods are described, e.g., in U.S. Publication No. US 2008/022004 A1.

The contacting step (i) is carried out under conditions adequate for binding of the biotinylated molecule to the biotin-binding molecule.

The term "conditions adequate for binding" refers to conditions of, for example, temperature, salt concentration, pH and protein concentration under which a biotinylated molecule can bind to the biotin-binding molecule. Exact binding conditions will vary depending upon the nature of the assay. It will be understood that the binding of the biotinylated molecule and the biotin-binding molecule need not bind at 100%. In some embodiments, essentially 100% is bound, i.e. at least 50%, at least is bound, at least 75% is bound, at least 80% is bound, at least 85% is bound or at least 95% is bound. Temperatures for binding can vary from 15°C to 37°C, but will preferably be between room temperature and about 30°C. The concentration of biotin-binding molecule or of the complex containing the biotin-binding molecule and the AQC-CTS in the binding reaction is no particularly limiting. In some embodiments, the concentration will vary, but will preferably be about 10 pM to 10 nM. In some embodiment, the biotin-binding molecule and, in particular, streptavidin is used at 0.5 mg/ml. In some embodiments, the binding reaction is carried out at a neutral pH. In some embodiment, the binding reaction is carried out at room temperature.

In a second step of the method for determination of biotinylated molecule using competitive assays according to the invention, the change in the intensity of the fluorescence emission by the AQC-CTC as a result of the contacting of step (i) is determined. It will be understood by the skilled person that the intensity is the only parameter of the fluorescence emission that is altered in the presence of the biotinylated molecule. Thus, in some embodiment, the determination in step (ii) does not involve the determination of the emission wavelength, the determination of the mean lifetime of the fluorescence or the determination of the anisotropy of the charge-transfer complex.

In order to carry out the second step of the method of the invention, the sample obtained after the contacting step (i) has been carried out is illuminated with a wavelength of light selected to give a detectable optical response, and observed with a means for detecting the optical response.

The skilled person will understand that the excitation wavelength required to obtain a fluorescence emission from the AQC-CTC will depend on the exact nature of the CTCs used in the method. The excitation wavelength can be determined by the skilled person by routine techniques. For instance, an approximate estimation of the cluster excitation and emission wavelengths can be determined by approximation by means of the Jellium model (see J.Calvo et al., Encyclopedia of Nanotechnology, Ed. by B. Bhushan, Springer Verlag, 2011, for example). This model predicts in a rather approximate manner the prohibited energy bandgap of the clusters and, therefore, the position of the emission bandgap thereof. The excitation bandgap of the clusters can in turn be predicted from the emission bandgap taking into account that the Stokes shift in clusters of a particular size is of approximate 50-100 nm. The following table, Table 1, shows the theoretical data for AQCs of Au or Ag according to this mode, i.e., the approximate excitation λ_{exc.}, and emission, λ_{em.}, wavelengths have been calculated with an error of ± 50 nm in AQCs of Au or Ag by means of said Jellium model: Eₑₘ = E_{F}/N^{1/3}; where Eₑₘ = emission energy; N= no. of atoms in the AQC; and E_{F} = Fermi level which is the same approximately 5.5 eV for gold and silver.

**Table 1.**

| Cluster | λ_{exc.}(nm) | λ_{em.} (nm) |
|---|---|---|
| A₂ | 200-250 | 300 |
| A₃ | 240-290 | 340 |
| A₄ | 270-320 | 370 |
| A₅ | 300-350 | 400 |
| A₆ | 325-375 | 425 |
| A₇ | 350-400 | 450 |
| A₁₀ | 400-450 | 500 |
| A₁₂ | 440-490 | 540 |
| A₁₅ | 475-525 | 575 |
| A₂₀ | 535-585 | 635 |
| A₂₅ | 580-630 | 680 |
| A₃₀ | 630-680 | 730 |
| A₄₀ | 700-750 | 800 |

Equipment that is useful for illuminating the dye compounds of the invention includes, but is not limited to, hand-held ultraviolet lamps, mercury arc lamps, xenon lamps, lasers and laser diodes. These illumination sources are optionally integrated into laser scanners, fluorescence microplate readers, standard or minifluorometers, or chromatographic detectors.

The presence of biotin or biotinylated molecules in the sample results in a decrease in the intensity of the fluorescence emitted by the CTCs with respect to eituer the fluorescence of the CTCs in the presence of a blank sample, i.e. a sample lacking any biotin or bbiotinylated molecule) or with respect to the fluorescence prior to the contacting with the sample.

The term "decrease in the fluorescence", as used herein, refers to a decrease in the intensity of the fluorescence. In some embodiments, the fluorescence in the sample in the presence of the biotinylated biomolecule or biotin decreases at least 5%, at least 10%, at least 15%, at least 20%, at least 25%, at least 30%, at least 40%, at least 50%, at least 60%, at least 70%, at least 80%, at least 90% or at least 100%, i.e. the fluorescence is indetectable with respect to the fluorescence in the absence of the biotinylated molecule (i.e. with respect to the fluorescence in a sample which does not contain any biotin or biotinylated molecule) or with respect to the fluorescence emission prior to the addition of the sample.

The optical response is optionally detected by visual inspection, or by use of any of the following devices: CCD cameras, video cameras, photographic films, laser-scanning devices, fluorometers, photodiodes, quantum counters, epifluorescence microscopes, scanning microscopes, flow cytometers, fluorescence microplate readers, or by means for amplifying the signal such as photomultiplier tubes. Where the sample is examined using a flow cytometer, examination of the sample optionally includes sorting portions of the sample according to their fluorescence response.

### Conjugates comprising AQC-CTCs and a biomolecule

The CTC-AQCs_described above may be further modified by the presence or ω-hydroxyacids ligands having the general formula HO-(CH₂)ₘ-COOH and ω-mercaptoacids having the general formula HS-(CH₂)ₚ-COOH). The hydroxyl and mercapto groups of the organic ligands interact with the metal atoms of the ACQs. Due to the amphiphilicty of the organic ligands, the carboxyl groups are exposed towards the outside the complex, being therefore available for conjugation with proteins, nucleic acids, and other biomolecules. These conjugates are particularly suited as fluorescent probe, biomarker or contrasting agent. These nanosystems can be used in biology systems both *in vitro* and *in vivo.*

In another embodiment, the invention relates to a conjugate comprising a biomolecule and a charge-transfer complex of at least two different size metal AQC, Mₙ and M'_{n'}, of general formula (I):

Mₙ⁺M'_{n'}⁻ (I),

wherein
the metals, M and M', of the metal AQCs are the same or different metals,
Mₙ, is the smaller AQC which is present in its oxidized form, Mₙ⁺,
M'_{n'}, is the larger AQC which is present in its reduced form, M'_{n'}⁻,
Mₙ⁺ and M'_{n'}⁻ are bound by electrostatic interactions,
n and n' are respectively the number of metal atoms of M and M', and
n is smaller than n'
wherein the conjugate further comprises ω-hydroxyacids and ω-mercaptoacids ligands attached to the atomic quantum clusters, Mₙ and M'_{n'} and wherein the biomolecule is attached to the ω-hydroxyacids and/or to the ω-mercaptoacid ligands.

In some embodiment the metals, M and M', of the metallic AQCs are selected from transition metals or combinations thereof, preferably the transition metals are selected from the group consisting of Au, Ag, Co, Cu, Pt, Fe, Cr, Pd, Ni, Rh and combinations thereof, more preferably they are selected from the group consisting of Au, Ag, Cu and combinations thereof, and more preferably the transition metals are selected from the group consisting of Au, Ag and the combination thereof.

Since the excitation and emission wavelengths of the fluorescence depend on the size of the AQCs, it is possible to select the excitation and emission wavelengths by directing the formation of AQCs of necessary sizes.

In a preferred embodiment, only one electron is transferred between the at least two AQCs, Mₙ and M'_{n'}, therefore resulting the ionic forms, Mₙ⁺, i.e. the oxidized form of Mₙ, and M'_{n'}⁻, the reduced form of M'_{n'}, wherein "+" is a positive charge and "-" is a negative charge.

In some embodiments, n and n' are between 2 and 309, between 2 and 102, between 2 and 55, or between 2 and 25 metal atoms. In some embodiments, the difference between n and n' is between 5 and 50 atoms.

In a further embodiment the difference between n and n' is between 5 and 50 atoms or between 5 and 25 atoms.

In some embodiments, the ω-hydroxyacid have the general formula HO-(CH₂)ₘ-COOH where m has a value between 2 and 30.

In some embodiments, the ω-mercaptoacid have the general formula HS-(CH₂)ₚ-COOH where p has a value between 2 and 30.

In some embodiment, m and p have a value between 10 and 20. In a particular embodiment m and p have a value of 15. In another particular embodiment m and p have a value of 11. The value of m and p can be different or the same. In the event that m and p are different the difference between them is less than 6 carbons, preferably the difference of the values of m and p is between 1 and 4. In a preferred embodiment m and p are the same. Wherein the at least two different types of organic ligands are selected from ω-hydroxyacid (HO-(CH₂)ₘ-COOH) and ω-mercaptoacid (HS-(CH₂)ₚ-COOH) ligands, the acid groups, -COOH, (or -COO⁻, if the salt of the corresponding acid is used) are directed towards the outer surface of the nanocompound and the -OH and -SH groups directed towards the inside, i.e. towards the ionized AQCs, Mₙ⁺ and M'_{n'}⁻, being bound, attached or coordinated to them.

In another embodiment the "organic ligands" that may be attached to the charge-transfer complex have other functional groups than hydroxyl, -OH, or mercapto, -SH groups, such as -NH₂, -NH-, -Cl, -PH₃, -SR, -OR, -NR₂, -NHR, -NR-, wherein R represents an organic group of a short hydrocarbon chain, C₁-C₄ capable of bound, attach or coordinate the AQCs or the ionized AQCs, Mₙ⁺ and M'_{n'}⁻. Is also possible exchanging the hydroxyl, -OH, or mercapto, -SH groups of the ω-hydroxyacid (HO-(CH₂)ₘ-COOH) and ω-mercaptoacid (HS-(CH₂)ₚ-COOH) ligands with these others, mentioned above that also interact with the metals of the AQCs.

Suitable biomolecules that can be incorporated in the conjugates according to the invention include any of the biomolecules mentioned above in the context of the competitive assays for the detection of biotinylated molecules using AQC-CTCs.

### Methods for detecting biomolecules using binding partners detectably labelled with a CTC-AQC

The conjugates comprising a biomolecule and a CTC-AQC can be used for the detection of any binding partner of the biomolecule by following the fluorescent emission of the AQC in a complex formed between the biomolecule and the binding partner. Thus, in another aspect, the invention relates to a method for detecting a target molecule in a sample comprising the steps of:
(i) contacting said sample with a conjugate comprising a conjugate according to the invention wherein the biomolecule of the conjugate binds specifically to said target molecule under conditions adequate for binding of the biomolecule to said binding partner and
(ii) detecting complex formation between the biomolecule and the target molecule.

The method comprises, in a first step, contacting the sample which is suspected to contain the target molecule with a conjugate comprising a charge transfer complex of an AQC and a biomolecule which is a binding partner for said target molecule, wherein the contacting step is carried out under conditions adequate for binding of the biomolecule to the moiety which specifically binds to said biomolecule.

In some embodiment the metals, M and M', of the metallic AQCs are selected from transition metals or combinations thereof, preferably the transition metals are selected from the group consisting of Au, Ag, Co, Cu, Pt, Fe, Cr, Pd, Ni, Rh and combinations thereof, more preferably they are selected from the group consisting of Au, Ag, Cu and combinations thereof, and more preferably the transition metals are selected from the group consisting of Au, Ag and the combination thereof.

Since the excitation and emission wavelengths of the fluorescence depend on the size of the AQCs, it is possible to select the excitation and emission wavelengths by directing the formation of AQCs of necessary sizes.

In a preferred embodiment, only one electron is transferred between the at least two AQCs, Mₙ and M'_{n'}, therefore resulting the ionic forms, Mₙ⁺, i.e. the oxidized form of Mₙ, and M'_{n'}⁻, the reduced form of M'_{n'}, wherein "+" is a positive charge and "-" is a negative charge.

In some embodiments, n and n' are between 2 and 309, between 2 and 102, between 2 and 55, or between 2 and 25 metal atoms. In some embodiments, the difference between n and n' is between 5 and 50 atoms.

In a further embodiment the difference between n and n' is between 5 and 50 atoms or between 5 and 25 atoms.

In some embodiments, the ω-hydroxyacid have the general formula HO-(CH₂)ₘ-COOH where m has a value between 2 and 30.

In some embodiments, the ω-mercaptoacid have the general formula HS-(CH₂)ₚ-COOH where p has a value between 2 and 30.

In some embodiment, m and p have a value between 10 and 20. In a particular embodiment m and p have a value of 15. In another particular embodiment m and p have a value of 11. The value of m and p can be different or the same. In the event that m and p are different the difference between them is less than 6 carbons, preferably the difference of the values of m and p is between 1 and 4. In a preferred embodiment m and p are the same. Wherein the at least two different types of organic ligands are selected from ω-hydroxyacid (HO-(CH₂)ₘ-COOH) and ω-mercaptoacid (HS-(CH₂)ₚ-COOH) ligands, the acid groups, -COOH, (or -COO⁻, if the salt of the corresponding acid is used) are directed towards the outer surface of the nanocompound and the -OH and -SH groups directed towards the inside, i.e. towards the ionized AQCs, Mₙ⁺ and M'_{n'}⁻, being bound, attached or coordinated to them.

In another embodiment the "organic ligands" that may be attached to the charge-transfer complex have other functional groups than hydroxyl, -OH, or mercapto, -SH groups, such as -NH₂, -NH-, -Cl, -PH₃, -SR, -OR, -NR₂, -NHR, -NR-, wherein R represents an organic group of a short hydrocarbon chain, C₁-C₄ capable of bound, attach or coordinate the AQCs or the ionized AQCs, Mₙ⁺ and M'_{n'}⁻. Is also possible exchanging the hydroxyl, -OH, or mercapto, -SH groups of the ω-hydroxyacid (HO-(CH₂)ₘ-COOH) and ω-mercaptoacid (HS-(CH₂)ₚ-COOH) ligands with these others, mentioned above that also interact with the metals of the AQCs.

Suitable pairs ofbiomolecules and target molecules are as shown in Table 2.

**Table 2: Representative binding pairs to be employed in the methods of the present invention.. IgG is an immunoglobulin; cDNA and cRNA are the antisense (complementary) strands used for hybridization.**

| Antigen/Hapten | Antibody |
|---|---|
| Biotin | Biotin-binding molecule (streptavidin, avidin, anti-biotin antibody) |
| IgG | Protein A or protein G |
| Folate | Folate binding protein |
| Toxin | Toxin receptor |
| Carbohydrate | Lectin or carbohydrate receptor |
| Peptide | Peptide receptor |
| Protein | Protein receptor or aptamer |
| Enzyme Substrate | Enzyme |
| DNA (RNA) | cDNA (cRNA) |
| Hormone | Hormone receptor |
| Ion | Chelator |

It will be appreciated that if the targaet molecule to be detected is any of the molecules mentioned in the left-hand column, then the biomolecule which is detectably labeled with the CTC-AQC is as defined in the right-hand column. Conversely, if the target molecule to be detected is any of the molecules mentioned in the right-hand column, then the biomolecule which is detectably labeled with a charge transfer complex of the AQC is as defined in the left-hand column.

In some embodiments, the biomolecule binds covalently to the target molecule. In some embodiments, the biomolecule binds non-covalently to the target molecule.

In some embodiments, the target molecule to be detected is an antigen or an hapten, in which case the biomolecule which is detectably labelled with the charge transfer complex of the AQC is an antibody which specifically binds to said antigen. In other embodiments, the target molecule to be detected is an antibody, in which case the biomolecule which is detectable labelled with the charge transfer complex of the AQC is an antigen which is recognised by said antibody.

In some embodiments, the target molecule to be detected is a nucleic acid, in which case the biomolecule which is detectably labelled with the charge transfer complex of the AQC is a nucleic acid which hybridizes specifically to said nucleic acid. The term "specifically hybridizing", as used herein, refers to conditions which allow hybridizing of two polynucleotides under high stringent conditions or moderately stringent conditions. "Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

Conditions suitable to promote binding of the biomolecule to the binding partner in the sample can be determined by those of skill in the art based on the teachings herein and the examples provided below. For example, antibody-antigen binding often depends on hydrophobic interactions (the so called hydrophobic bonds); thus, high salt concentrations, such as in the molar range can be used to reduce nonspecific binding and increase specific antigen- antibody binding.

For biological applications, the contacting step of the sample and binding partner for the target biomolecule is carried out in an aqueous, mostly aqueous or aqueous-miscible solution prepared according to methods generally known in the art. The exact concentration of detectable labeled binding partner is dependent upon the experimental conditions and the desired results, but typically ranges from about one nanomolar to one millimolar or higher. The optimal concentration is determined by systematic variation until satisfactory results with minimal background fluorescence are accomplished.

In a second step, the method for detecting a target molecule using the conjugates according to the invention involves detecting complex formation between the biomolecule which is detectably labelled with the charge transfer complex of the AQC and the target molecule.

The detection of complex formation can be carried out by detecting the fluorescence emitted by the charge transfer complex of the AQC upon excitation at a suitable wavelength.

Typically, the detection of the complex is carried out after separating the complex from any unbound detectably labeled biomolecule still present in the sample. Different methods are available to the skilled person for separating the complex from any unbound detectable labeled biomolecule still present in the sample.

In some embodiments, the target molecule is immobilized in a support, which allows the recovery of the complex formed with the detectably labeled biomolecule by separating the support from the sample. Suitable supports which can be used for the immobilization of the binding partner include, without limitation, any of those defined above in the context of the competitive assays according to the invention.

In some embodiments, the separation of the complex from any unbound detectably labeled binding partner is carried out using a second binding partner for the target molecule, which is different from the biomolecule which is detectably labeled with the CTC-AQC. This allows the recovery of a ternary complex comprising the detectably labeled biomolecule, the target molecule and the second binding partner. For instance, if the target molecule to be detected is a polypeptide and the detectably labeled biomolecule is an antibody, the complex between the polypeptide and the detectably labeled biomolecule can be separated using a second antibody specific for the target molecule and which recognizes a different region of the target molecule so that avoid that the binding of the second antibody to the target molecule is prevented by steric hindrance. In some embodiments, the target molecule contains a tag. Suitable tags that can be used in the present invention include, without limitation, peptide-based tags. The tag is generally placed at the amino- or the carboxyl-terminus of the polypeptide. Various tag polypeptides and their respective antibodies are well known in the art. Examples include poly-histidine (poly-his) or poly-histidine-glycine (poly-his-gly) tags; the flu HA tag polypeptide and its antibody 12CA5 (Field et al., Mol Cell Biol, 1988;8:2159-2165); the c-myc tag and the 8F9, 3C7, 6E10, G4, B7 and 9E10 antibodies thereto (Evan et al., Molecular and Cellular Biology 1985;5:3610-3616); the Herpes Simplex virus glycoprotein D (gD) tag and its antibody (Paborsky et al., Protein Engineering 1990;3:547-553). Other tag polypeptides include the Flag-peptide (Hopp et al., BioTechnology 1988;6:1204-1210); the KT3 epitope peptide [Martin et al., Science 1993;255:192-194); tubulin epitope peptide (Skinner et al., J Biol Chem 1991;266:15163-15166); and the T7 gene 10 protein peptide tag (Lutz-Freyermuth et al., Proc Natl Acad Sci USA 1990,;87:6393-6397). In a preferred embodiment, the purification tag is a polyhistidine tag. In a still more preferred embodiment, the purification tag is a hexahistidine tag.

In some embodiments, after the complex has been separated from any unbound detectably labeled biomolecule, the complex is washed at least once in order to remove excess labeled biomolecule. The washing steps may be carried out in any appropriate way depending on the biomolecule and the binding partners. The sample may be washed with any suitable medium, e.g. a a buffer such as PBS. The medium may contain a detergent such as Tween20. It may be washed for any suitable time, e.g. 1 to 30 minutes or 3 to 10 minutes for each wash. Washing may include gentle shaking or rocking of the carrier of said cell. The washing temperature is such that the cell can survive and binding is not disrupted. For example, it can be between 20 and 45°C or between 30 and 40°C. Typically, it is about 37°C or room temperature. Suitable protocols for washing complexes of the biomolecule and binding partner are well known in the art.

The detection of the complex between the detectably labeled biomolecule and the target molecule involves detecting the fluorescence emission of the AQC-CTC associated to the target molecule. If a fluorescence emission is detected, this means that a detectable amount of the biomolecule has been recovered together with the target molecule in step (ii) and, therefore, that the sample contained the target molecule.

In some embodiments, detection of the target molecules can be carried out by Western blot, in which case the target molecules are separated by gel electrophoresis and transferred to a blotting membrane and contacted with the detectably labelled biomolecule. Alternatively, in some embodiments, the target molecules are contacted with the biomoelcule and then separated under conditions wherein the interaction of the biomolecule and the target molecule is preserved. The complex can then be detected by measuring the fluorescence within the support used for the separation of the complex. Separation can be carried out by any suitable means such as electrophoresis or chromatography. In some embodiments, the separation of the complex is carried out by gel electrophoresis, in which case the detection is carried out by in-gel fluorescence detection.

Suitable means and conditions for exciting the charge transfer complex of the AQC, as well as means and conditions for detecting the fluorescence emission of the charge transfer complex of the AQC have been described in the context of the competitive methods according to the invention and are equally applicable to the present inventive method.

### Methods for the preparation of the conjugate comprising a biomolecule and a charge transfer complex of an AQC

In another aspect, the invention relates to a method for preparing a conjugate comprising a biomolecule and a charge transfer complex of the AQCs. The charge transfer complex of the AQCs are stabilised by ω-hydroxyacids and ω-mercaptoacids ligands wherein the AQCs are bound to the ω-hydroxy and ω-mercapto groups of the organic ligands, leaving the carboxy groups exposed which are then available for coupling with a molecule of interest.

Thus, in another aspect, the invention relates to a method for preparing a conjugate of a charge-transfer complex and a biomolecule according to the invention said method comprising reacting a charge transfer complex of an AQC which has been functionalized on its surface with a first reactive group with a biomolecule containing second reactive groups which can react with the first reactive group.

The charge transfer complex of the AQC contain organic acids which contain carboxyl groups which are exposed towards the outside of the ACQ. Accordingly, in some embodiments, it is possible to directly activate the carboxylic group in order to introduce a reactive group that can react with a corresponding reactive group in the biomolecule. Representative reactive groups that can be incorporated in the organic acids of the charge transfer complex of the AQCs include, without limitation, carboxylic acid groups, carboxylic acid active esters (e.g., N-hydroxysuccinimide esters), maleimide groups, reactive carbamate and thiocarbamate groups, and α-haloacetamide groups (-NH-C(-O)-CH2-X). Other suitable functional groups include groups that are capable of coupling the cycloaddition (e.g., dienes and dienophiles to provide 4+2 cycloaddition products, and acetylenes and azides (click chemistry)). In one embodiment, the reactive group is a carboxylic acid group (-CO2H) or its active esters (e.g., N-hydroxysuccinimide ester).

Carboxylic acid groups and carboxylic acid active esters are reactive toward amino groups including the amino group of lysine residues in proteins and peptides, and primary amino groups introduced into oligonucleotide probes (-C(-O)-NH- linkage). Maleimide groups are reactive to sulfhydryl groups native to or introduced into protein, peptide, and oligonucleotides (-N[C(-O)CH2CHC(-O)]-S- linkages). Reactive carbamate and thiocarbamate groups are reactive toward amino groups to provide urea (-NH-C(-O)-NH-) and thiourea (-NH-C(-S)-NH-) linkages. α-Haloacetamide groups are reactive toward thiol groups to provide -NH-C(-O)-CH2-S- linkages. Functional groups capable of conjugation through cycloaddition include dienes (e.g., furans) and dienophiles (e.g., alkenes and alkynes) that react to form 4+2 cycloaddition linkages. The linker arm can be modified to include either a diene or dienophile reactive toward a dienophile and diene, respectively, native to or incorporated into the complementary reactive material (e.g., biomolecule). Click chemistry can also be utilized for conjugation. The linker arm can be modified to include either a suitable acetylene (e.g., H-C=C-R) or azide (e.g., R'-N-N+-N-) reactive toward an azide or acetylene, respectively, native to or incorporated into the complementary reactive material (e.g., biomolecule).

In some embodiments, conjugation is carried out by reacting the carboxylic acid group naturally occurring in the organic acid or an active ester thereof with an amino group found in the biomolecule. Examples of activated esters include, but are not limited to, N- hydroxy succinimidyl ester, N-hydroxy sulfosuccinimidyl ester, p-sulfotetrafluorophenol ester, pentafluorophenyl esters, tetrafluorophenyl esters, p-nitrophenyl esters, 2,4-dinitrophenyl ester, 4- nitrophenyl ester, 3-Hydroxy-3,4-dihydro-4-oxo-1,2,3-benzotriazine (HODhbt), carboxylic acids activated using common carbodiimides such as but not limited to diisopropylcarbodiimide (DIPCDI), ?,?'-dicyclohexylcarbodiimide (DCC), 1-ethyl-3-(3'-dimethylaminopropyl)carbodiimide hydrochloride (EDC); and carboxylic acids activated with an uronium salt or a phosphonium salt, such as but not limited to 0-Benzotriazole-N,N,N',N'-tetramethyluronium hexafluorophosphate (HBTU), O-(Benzotriazol-1 -yl)- 1,1,3,3-tetramethyluronium tetrafluoroborate (TBTU), 2-(6-Chloro-1H-benzotriazole-1 -yl)-1, 1,3,3- tetramethylaminium hexafluorophosphate (HCTU), (2-(6-Chloro-1H-benzotriazole-1 -yl)-1, 1,3,3- tetramethylaminium tetrafluoroborate) (TCTU), 2-(1H-7-Azabenzotriazol-1-yl)-1,1,3,3-tetramethyl uronium hexafluorophosphate (HATU), 1-benzotriazolyoxytris-(dimethylamino)phosphonium hexafluorophosphate (BOP) benzotriazol-1 -yl-oxytripyrrolidinophosphonium hexafluorophosphate (PYBOP); or combinations thereof.

Wherein the biomolecule is a polypeptide, reactive amino groups appear naturally in the lysine residues, thereby allowing the conjugation of the biomolecule without further modification. Wherein the biomolecule is a polynucleotide, in one embodiment, the biomolecule can modified by the incorporation of an amino group. This is usually carried out during synthesis of the polynucleotide using an amino-modified oligonucleotide. Methods for introducing an amino group into an polynucleotide of choice have been described, i.e, in W02011131693, Kojima N. and Komatsu Y., (Curr. Protoc. Nucleic Acid Chem. 2012 Mar;Chapter 4:Unit 4.48.1-23. doi: 10.1002/0471142700.nc0448s48) and Vasilyeva SV et al. (Nucleosides Nucleotides Nucleic Acids. 2011; 30: 753-67). Wherein the biomolecule is an oligosaccharide or a polysaccharide, amino groups can be inserted by reductive amination as described, e.g. in US5306492 and in Porro et al., (Mol. Immunol., 1985, 22:907-919).

The reactive group in the charge transfer complex of AQC can be directly coupled to the carboxy groups in the organic acids or, alternatively, connected to this group by a spacer group or linker group, in order to avoid that the reaction between the reactive groups in the ACQ and in the biomolecule is prevented by steric hindrance. Similarly, the biomolecule which is to be conjugated with the reactive group may also contain spacer groups between the backbone of the biomolecule in order to prevent that reaction. In some embodiments, both the reactive group attached to the biomolecule and the corresponding reactive group attached to the ACQ via the organic acid contain a linker group. Suitable linker moieties include from one to about 50 atoms selected from carbon, nitrogen, oxygen, hydrogen, and halogen. Representative groups include alkylene groups (e.g., -(CH₁)ₙ-, where n is 1-12), phenylene groups (e.g., o-, m-, and p-C₆H₄-), and alkylene oxide groups (e.g., ethylene oxide, -(CH₂CH₂O)ₘ-, where m is 1-5). Other suitable groups include -C(=A₁)-L₁-, -C(=A₁)NH-L₁-, -C(=A₁)NH-L₁-NH-, wherein A₁ is selected from O and S, and L₁ is any of the above linker groups.

The activated charge transfer complex of the AQC can be provided as a protected form using a protecting group. Examples of protecting groups can be found in T. W. Greene and P. G. Wuts, PROTECTIVE GROUPS IN ORGANIC CHEMISTRY, (Wiley, 2nd ed. 1991), Beaucage and Iyer, Tetrahedron 48:2223-2311 (1992), and Harrison and Harrison et al., COMPENDIUM OF SYNTHETIC ORGANIC METHODS, Vols. 1-8 (John Wiley and Sons. 1971-1996). Representative amino protecting groups include formyl, acetyl, trifluoroacetyl, benzyl, benzyloxycarbonyl (CBZ), tert-butoxycarbonyl (Boc), trimethyl silyl (TMS), 2-trimethylsilyl-ethanesulfonyl (SES), trityl and substituted trityl groups, allyloxycarbonyl, 9-fluorenylmethyloxycarbonyl (FMOC), nitro-veratryloxycarbonyl (NVOC) and the like (see also, Boyle, A. L. (Editor), CURRENT PROTOCOLS IN NUCLEIC ACID CHEMISTRY, John Wiley and Sons, New York, Volume 1, 2000). Representative hydroxy protecting groups include those where the hydroxy group is either acylated or alkylated such as benzyl and trityl ethers as well as alkyl ethers, tetrahydropyranyl ethers, trialkylsilyl ethers and allyl ethers. Additionally, hydroxy groups can be protected by photoremovable groups such as α-methyl-6-nitropiperonyloxycarbonyl (McGall, G. H. and Fidanza, J. A., Photolithographic synthesis of high-density olignucleotide arrays, in DNA ARRAYS METHODS AND PROTOCOLS, Edited by Rampal J. B., METHODS IN MOLECULAR BIOLOGY, 170:71-101 (2001), Humana Press, Inc., NY; Boyle, Ann L. (Editor), Current Protocols in Nucleic Acid Chemistry, John Wiley and Sons, New York, Volume 1, 2000).

### Methods for labeling an intracellular component

In another embodiment, the invention relates to a method for *in vivo* labeling an intracellular component comprising: contacting one or more intracellular components with a conjugate comprising an AQC-CTC and a biomolecule which binds specifically to said intracellular component thereby allowing detection of one or more intracellular components by microscopy.

In one embodiment, the biomolecule is a polypeptide which binds specifically to a polypeptide expressed at one or more intracellular compartments, in which case the conjugates comprising the AQC-CTC according to the invention can be used for immunohistochemistry.

In another embodiment, the biomolecule is a nucleic acid which specifically hibrdises with a nucleic acid sequence of interest, in which case the conjugates comprising the AQC-CTC according to the invention can be used for fluorescent in situ hybridyzation (FISH). Typically, FISH methods involve the following steps: (a) fixing the tissue or other biological material under investigation to a support (e.g., glass slide or wall of a micro titer well), (b) treatment of the tissue or material to increase accessibility of FISH probe to target nucleic acid, (c) contacting the tissue or material containing the target nucleic acid with probes to form specific hybridization complexes, (d) post hybridization washes of the complexes to selectively remove probes that are not specifically hybridized to the target, and (e) detection of probes that have formed hybridization complexes with target nucleic acid molecules. Such methods are described in a number of sources, including: Gall and Pardue, (1981) Methods of Enzymology 21:470-480; Henderson, (1982) International Review of Cytology, 76: 1-46; and Angerer, et al., (1985) in Genetic Engineering: Principles and Methods (Setlow and Hollaender, Eds.) vol. 7, pp. 43-65, Plenum Press, New York.

In some embodiments, the biomolecule is selected from the group consisting of a polypeptide and a nucleic acid. In a still more preferred embodiment, the polypeptide is an antibody.

The conjugate is contacted with the sample in any way that facilitates contact between the dye compound and the sample components of interest. Typically, the conjugate or a solution containing the conjugate is simply added to the sample. Certain binding partners of the invention, tend to be impermeant to membranes of biological cells, and once inside viable cells are typically well retained. Treatments that permeabilize the plasma membrane, such as electroporation, shock treatments or high extracellular ATP can be used to introduce the binding partner into cells. Alternatively, the binding partner can be physically inserted into cells, e.g. by pressure microinjection, scrape loading, patch clamp methods, or phagocytosis.

Any tissue sample from a subject may be used. Examples of tissue samples that may be used include, but are not limited to, breast, prostate, ovary, colon, lung, endometrium, stomach, salivary gland or pancreas. The tissue sample can be obtained by a variety of procedures including, but not limited to surgical excision, aspiration or biopsy. The tissue may be fresh or frozen. In one embodiment, the tissue sample is fixed and embedded in paraffin or the like. The tissue sample may be fixed (i.e. preserved) by conventional methodology [See e.g., "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C. By way of example, neutral buffered formalin, Bouin's or paraformaldehyde, may be used to fix a tissue sample

Detection of the CTC-AQC associated with the cellular component can be carried out using of any of the following devices: CCD cameras, video cameras, photographic films, laser-scanning devices, fluorometers, photodiodes, quantum counters, epifluorescence microscopes, scanning microscopes, flow cytometers, fluorescence microplate readers, or by means for amplifying the signal such as photomultiplier tubes. Where the sample is examined using a flow cytometer, examination of the sample optionally includes sorting portions of the sample according to their fluorescence response.

### Further aspects of the invention:

1. A complex containing a biotin-binding molecule and a charge-transfer complex of at least two different size metal atomic quantum clusters (AQCs), Mₙ and M'_{n'}, of general formula (I):

   Mₙ⁺M'_{n'}⁻ (I),

   wherein
   the metals, M and M', of the metal AQCs are the same or different metals,
   Mₙ, is the smaller AQC which is present in its oxidized form, Mₙ⁺,
   M'_{n'}, is the larger AQC which is present in its reduced form, M'_{n'}⁻,
   Mₙ⁺ and M'_{n'}⁻ are bound by electrostatic interactions,
   n and n' are respectively the number of metal atoms of M and M', and
   n is smaller than n'
   wherein the biotin-binding molecule and the charge-transfer complex are not covalently bound.
2. The complex according to aspect 1 wherein the biotin-binding molecule is streptavidin or a functionally equivalent variant thereof.
3. The complex according to aspects 1 or 2 wherein the metals M and M' are independently selected from transition metals or combinations thereof.
4. The complex according to any of aspect 3 wherein the metals M and M' are independently selected from the transition metals Au, Ag, Cu and combinations thereof, and more preferably Au, Ag and the combination thereof.
5. The complex according to any of aspects 1 to 4 wherein n and n' are between 2 and 309, between 2 and 102, between 2 and 55, or between 2 and 25 metal atoms.
6. The complex according to any of aspects 1 to 5 wherein the difference between n and n' is between 5 and 50 atoms.
7. The complex according to any of aspects 1 to 6 wherein the charge-transfer complex further comprise ω-hydroxyacids andr ω-mercaptoacids ligands attached to the atomic quantum clusters, Mₙ and M'_{n'}.
8. The complex according to aspect 7 wherein the ω-hydroxyacids have the general formula (HO-(CH₂)ₘ-COOH) wherein m has a value between 2 and 30 and/or wherein the ω-mercaptoacid have the general formula HS-(CH₂)ₚ-COOH ligands wherein p has a value between 2 and 30.
9. A kit-of-parts containing, together or separately, a charge-transfer complex of at least two different size metal atomic quantum clusters (AQCs), Mₙ and M'ₙ and a biotin-binding molecule wherein the charge-transfer complex of at least two different size metal atomic quantum clusters (AQC) has the formula I as defined in aspect 1 and wherein M and M', of the metal AQCs are the same or different metals, Mₙ, is the smaller AQC which is present in its oxidized form, Mₙ⁺, M'_{n'}, is the larger AQC which is present in its reduced form, M'_{n'}⁻, Mₙ⁺ and M'_{n'}⁻ are bound by electrostatic interactions, n and n' are respectively the number of metal
10. A method for the detection of a biotinylated molecule in a sample which comprises the steps of:
   (i) contacting said sample with a complex according to any of aspects 1 to 8 under conditions adequate for binding of the biotinylated molecule to the biotin-binding molecule in the complex and
   (ii) detecting the change in the intensity of the fluorescence emission by the CTC following the contacting of step (i) in response to the excitation of the sample at the excitation wavelength of the CTC
   wherein a decrease in the fluorescence intensity emitted by the CTC afer the contacting step is indicative of the presence in the sample of a biotinylated molecule.
11. The method according to aspect 10 wherein the biotinylated molecule is a nucleic acid, a polypeptide or a polysaccharide.
12. The method according to aspects 10 or 11 wherein the biotin-binding molecule is provided as a complex with a biotin analogue which shows an affinity towards the biotin-binding molecule which is lower than that of biotin.
13. A conjugate comprising a biomolecule and a charge-transfer complex of at least two different size metal AQC, Mₙ and M'_{n'}, of general formula (I):

   Mₙ⁺M'_{n'}⁻ (I),

   wherein
   the metals, M and M', of the metal AQCs are the same or different metals,
   Mₙ, is the smaller AQC which is present in its oxidized form, Mₙ⁺,
   M'_{n'}, is the larger AQC which is present in its reduced form, M'_{n'}⁻,
   Mₙ⁺ and M'_{n'}⁻ are bound by electrostatic interactions,
   n and n' are respectively the number of metal atoms of M and M', and
   n is smaller than n'.
   wherein the conjugate further comprises ω-hydroxyacids and ω-mercaptoacids ligands attached to the atomic quantum clusters, Mₙ and M'_{n'} and wherein the biomolecule is attached to the ω-hydroxyacids and/or to the ω-mercaptoacids ligands.
14. The conjugate according to aspect 13 wherein the biomolecule is selected from the group consisting of a nucleic acid, a polysaccharide and a polypeptide.
15. The conjugate according to aspects 13 or 14 wherein the metals, M and M' are independently selected from transition metals or combinations thereof.
16. The conjugate according to any of aspects 13 to 15 wherein the metals M and M', are independently selected from the transition metals Au, Ag, Cu and combinations thereof, and more preferably Au, Ag and the combination thereof.
17. The conjugate according to any of aspects 13 to 16 wherein n and n' are between 2 and 309, between 2 and 102, between 2 and 55, or between 2 and 25 metal atoms.
18. The conjugate according to any of aspects 13 to 17 wherein the difference between n and n' is between 5 and 50 atoms.
19. A method for the preparation of a conjugate according to any of aspects 13 to 18 comprising reacting a charge transfer complex of an AQC which has been functionalized on its surface with a first reactive group with a biomolecule containing groups which can react with the first reactive group.
20. The method according to aspect 19 wherein the group in the biomolecule have been introduced by pre-functionalization of the biomolecule pair prior to the reacting step.
21. The method according to aspect 19 or 20 wherein the first reactive group is an activated carboxyl group.
22. The method according to aspect 21 wherein the functional group of the first member of the binding pair is an activated hydroxyl group or an activated amino group.
23. A method for detecting a biomolecule in a sample comprising the steps of:
   (i) contacting said sample with a conjugate comprising a conjugate according to any of aspects 9 to 12 wherein the biomolecule binds specifically to said target molecule under conditions adequate for binding of the biomolecule to said target molecule and
   (ii) detecting complex formation between the biomolecule and the target molecule.
24. The method according to aspect 23 wherein the biomolecule is a first polypeptide and wherein the binding partner is selected from the group consisting of a second polypeptide and an aptamer.
25. The method according to aspect 24 wherein the second polypeptide is antibody.
26. The method according to aspect 23 wherein the biomolecule is a first nucleic acid and wherein the binding partner is a second nucleic acid which hybridizes specifically to the first nucleic acid.
27. The method according to aspect 23 wherein the biomolecule is a polysaccharide and the binding partner is a lectin or wherein the biomolecule is a lectin and the binding partner is a polysaccharide.
28. The method according to any of aspects 23 to 27 wherein the biomolecule is present in a cell, bacteria, virus, or yeast cell, or is immobilized on a polymer, polymeric membrane or polymeric particle.
29. A method for detecting an intracellular component comprising: contacting one or more intracellular components with a conjugate comprising an AQC-CTC and a binding partner which specifically binds to said intracellular component thereby allowing detection of one or more intracellular component by microscopy.
30. The method according to aspect 29 wherein the binding partner is selected from the group consisting of a polypeptide and a nucleic acid.
31. The method according to aspect 31 wherein the polypeptide is an antibody.

The invention is described by way of the following examples that are to be construed as merely illustrative and not limitative of the scope of the invention.

### EXAMPLES

### Reagents

Streptavidin (from *Streptomyces avidinii,* affinity purified, ≥13U/mg) and oligo-biotin (36bp oligonucleotide-biotin,
AACACCGCAGCATGTCAAGATCACACATTTTGGGCG[bTNtG]) (SEQ ID NO:2)
were purchased from Sigma-aldrich and used without any further purification, preparing stock solutions as recommended by literature and the manufacturer: streptavidin 0.5 mg/ml in water and oligo-biotin 1nM in TE buffer (tris-EDTA buffer solution, pH=8.0, Fluka).

AQC-CTC were synthesized by standard protocol. An aqueous solution of 16-hydroxypalmitic acid (98%, Aldrich) (2 ml, 10 mg/ml) was mixed with an aqueous solution of 16-mercaptopalmitic acid (90%, Aldrich) (0.622 ml, 10 mg/mL) with vigorous stirring in 5.4 ml of water and the necessary volume of tetrabutylammonium hydroxide (40% in water, Fluka) until producing a basic mean. A volume of 400 µL of HAuCl₄.3H₂O (Au (III) chloride hydrate, metal base at 99.999%, Aldrich) (5.8 mg/ml) solution was added to the resulting solution of nanosomes with subsequent reduction by means of adding 400 µL of a NaBH₄ (96%, Aldrich) solution (0.05 M). This solution was stirred at 35° C for 1 hour. In order to get the AQC-CTC, this solution was ultracentrifuged at 90,000 rpm for 1h in a Beckman Airfuge® Air-Driven ultracentrifuge, obtaining the AQC-CTC as the supernatant of the separation.

### Example 1. Biotinylated oligonucleotide detection with streptavidin fluorescence enhancement of AQC-CTC.

As table 1 shows three different solutions were prepared, containing 15 µl of AQC-CTC (150 mg/l), 55 µL of streptavidin solution (0.5 mg/ml in water) and increasing amounts of Oligo-biotin solution (1 nM in TE buffer). After 1h incubation fluorescence of each sample was measured.

**Table 3**

| Solution | V_{AQC}/µl | V_{Oligo}/µl | mmol Oligo | Vₛₜᵣₑₚ/µl |
|---|---|---|---|---|
| A | 0.15 | 0 | 0 | 55 |
| B | 0.15 | 0.10 | 0.1 | 55 |
| C | 0.15 | 0.15 | 0.5 | 55 |

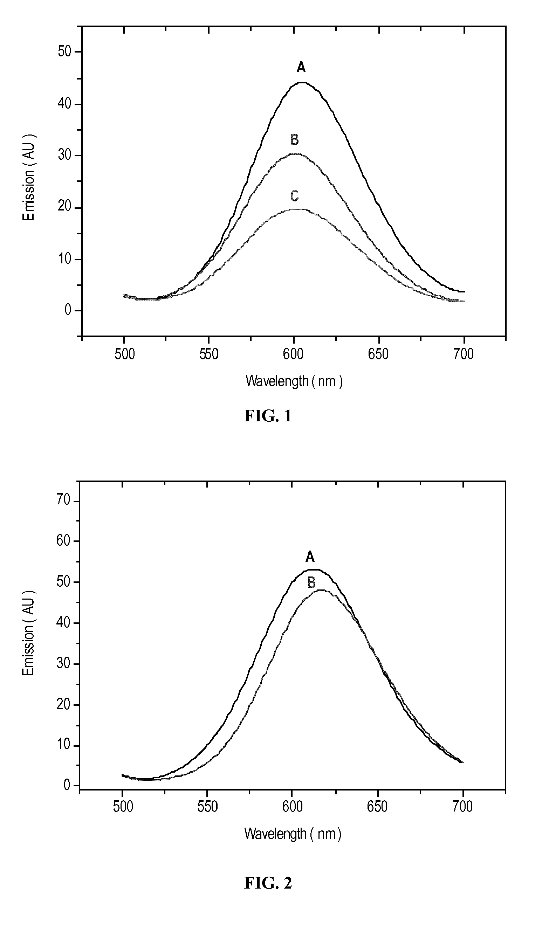

Figure 1 shows emission spectra at 250 nm of excitation (measured with a Cary Eclipse Varian Fluorescence spectrophotometer in a quartz cell of 3x3 mm of light path and 45 µl of volume) of the different solutions: (A) AQC-CTC with streptavidin, (B) AQC-CTC with streptavidin and 0.1 mmol of oligo-biotin and (C) AQC-CTC with streptavidin and 0.5 mmol of oligo-biotin. This spectra show an emission maximum at 600 nm that decrease with the addition of the Oligo-biotin, being the emission maximum lower as the amount of oligo-biotin increased.

### Example 2. Biotinylated oligonucleotide detection with streptavidin/HABA fluorescence enhancement/quenching of AQC-CTC.

As table 2 shows, two solutions were prepared adding 0.25 µl of HABA solution (10mM) and 0.5 µl the AQC-CTC solution (aprox. 150 mg/l) on the specified volume of streptavidin solution (0.5 mg/ml). Then, 0.5 µl of oligo-biotin solution (1 nM) were added to the solution B and both samples were incubated during 30 min.

**Table 4.**

| | Vₛₜᵣₑₚₜ/µl | V_{HABA}/µl | V_{Oligo}/µl | V_{AQC}/µl |
|---|---|---|---|---|
| A | 49.26 | 0.25 | 0 | 0.5 |
| B | 48.76 | 0.25 | 0.5 | 0.5 |

Figure 2 shows emission spectra at 250 nm of excitation (measured with a Cary Eclipse Varian Fluorescence spectrophotometer in a quartz cell of 3x3 mm of light path and 45 µl of volume) of the different solutions, (A) AQC-CTC with HABA and streptavidin and (B) AQC-CTC with HABA, streptavidin and 0.5 µl of oligo-biotin. As shown in figure 2, the addition of oligo-biotin results in a decrease on the emission maximum intensity, due to the interaction between the oligo-biotin and the streptavidin.

## Claims

1. A complex containing a biotin-binding molecule and a charge-transfer complex (CTC) of at least two different size metal atomic quantum clusters (AQCs), Mₙ and M'_{n'}, of general formula (I):
Mₙ⁺M'_{n'}⁻ (I),
wherein
the metals, M and M', of the metal AQCs are the same or different metals,
Mₙ, is the smaller AQC which is present in its oxidized form, Mₙ⁺,
M'_{n'}, is the larger AQC which is present in its reduced form, M'_{n'}⁻,
Mₙ⁺ and M'_{n'}⁻ are bound by electrostatic interactions,
n and n' are respectively the number of metal atoms of M and M', and
n is smaller than n'
wherein the biotin-binding molecule and the charge-transfer complex are not covalently bound.

2. The complex according to claim 1 wherein the biotin-binding molecule is streptavidin or a functionally equivalent variant thereof.

3. The complex according to claims 1 or 2 wherein
(i) M and M' are independently selected from transition metals or combinations thereof, preferably Au, Ag, Cu and combinations thereof, and more preferably Au, Ag and the combination thereof,
(ii) n and n' are between 2 and 309, between 2 and 102, between 2 and 55, or between 2 and 25 metal atoms and/or
(iii) wherein the difference between n and n' is between 5 and 50 atoms.

4. The complex according to any of claims 1 to 3 wherein the charge-transfer complex further comprise ω-hydroxyacids andr ω-mercaptoacids ligands attached to the atomic quantum clusters, Mₙ and M'_{n'}., preferably wherein the ω-hydroxyacids have the general formula (HO-(CH₂)ₘ-COOH) wherein m has a value between 2 and 30 and/or wherein the ω-mercaptoacid have the general formula HS-(CH₂)ₚ-COOH ligands wherein p has a value between 2 and 30.

5. A kit-of-parts containing, together or separately,
(i) a charge-transfer complex of at least two different size metal atomic quantum clusters (AQCs), Mₙ and M'ₙ and a biotin-binding molecule wherein the charge-transfer complex of at least two different size metal atomic quantum clusters (AQC) has the formula I as defined in claim 1 and wherein M and M', of the metal AQCs are the same or different metals, Mₙ, is the smaller AQC which is present in its oxidized form, Mₙ⁺, M'_{n'}, is the larger AQC which is present in its reduced form, M'_{n'}⁻, Mₙ⁺ and M'_{n'}⁻ are bound by electrostatic interactions, n and n' are respectively the number of metal atoms and
(ii) a biotin-binding molecule.

6. A method for the detection of a biotinylated molecule in a sample which comprises the steps of:
(i) contacting said sample with a complex according to any of claims 1 to 4 under conditions adequate for binding of the biotinylated molecule to the biotin-binding molecule in the complex and
(ii) detecting the change in the intensity of the fluorescence emission by the ACQ following the contacting of step (i) in response to the excitation of the sample at the excitation wavelength of the ACQ
wherein a decrease in the fluorescence intensity emitted by the ACQ after the contacting step is indicative of the presence in the sample of a biotinylated molecule.

7. The method according to claim 6 wherein the biotinylated molecule is a nucleic acid, a polypeptide or a polysaccharide.

8. The method according to claims 6 or 7 wherein the biotin-binding molecule is provided as a complex with a biotin analogue which shows an affinity towards the biotin-binding molecule which is lower than that of biotin, preferably, wherein the biotin analogue 4-hydroxyazobenzene-2-carboxylic acid (HABA).

9. A conjugate comprising a biomolecule and a charge-transfer complex of at least two different size metal AQC, Mₙ and M'_{n'}, of general formula (I):
Mₙ⁺M'_{n'}⁻ (I),
wherein
the metals, M and M', of the metal AQCs are the same or different metals,
Mₙ, is the smaller AQC which is present in its oxidized form, Mₙ⁺,
M'_{n'}, is the larger AQC which is present in its reduced form, M'_{n'}⁻,
Mₙ⁺ and M'_{n'}⁻ are bound by electrostatic interactions,
n and n' are respectively the number of metal atoms of M and M', and
n is smaller than n'.
wherein the conjugate further comprises ω-hydroxyacids and ω-mercaptoacids ligands attached to the atomic quantum clusters, Mₙ and M'_{n'} and wherein the biomolecule is covalently attached to the ω-hydroxyacids and/or to the ω-mercaptoacids ligands.

10. The conjugate according to claim 9 wherein the biomolecule is selected from the group consisting of a nucleic acid, a polysaccharide and a polypeptide.

11. The conjugate according to claims 9 or 10 wherein
(i) M and M' are independently selected from transition metals or combinations thereof, preferably Au, Ag, Cu and combinations thereof and more preferably Au, Ag and the combination thereof,
(ii) n and n' are between 2 and 309, between 2 and 102, between 2 and 55, or between 2 and 25 metal atoms and/or
(iii) the difference between n and n' is between 5 and 50 atoms.

12. The conjugate according to any of claims 9 to 11 wherein the ω-hydroxyacids have the general formula (HO-(CH₂)ₘ-COOH) wherein m has a value between 2 and 30 and/or wherein the ω-mercaptoacid have the general formula HS-(CH₂)ₚ-COOH ligands wherein p has a value between 2 and 30.

13. A method for the preparation of a conjugate according to any of claims 9 to 12 comprising reacting a charge transfer complex of an AQC which has been functionalized on its surface with a first reactive group with a biomolecule containing groups which can react with the first reactive group.

14. A method for detecting a target molecule in a sample comprising the steps of:
(i) contacting said sample with a conjugate comprising a conjugate according to any of claims 9 to 12 wherein the biomolecule binds specifically to said target molecule under conditions adequate for binding of the biomolecule to said target molecule and
(ii) detecting complex formation between the biomolecule and the target molecule.

15. The method according to claim 14 wherein if the biomolecule is a first polypeptide, then the binding partner is selected from the group consisting of a second polypeptide and an aptamer and wherein if the biomolecule is a first nucleic acid, then the binding partner is a second nucleic acid which hybridizes specifically to the first nucleic acid.

16. A method for detecting an intracellular component comprising: contacting one or more intracellular components with a conjugate comprising an AQC-CTC according to any of claims 9 to 12 wherein the biomolecule is a binding partner which specifically binds to said intracellular component thereby allowing detection of one or more intracellular component by microscopy.


SEQUENCE LISTING

<110>  NANOGAP SUB NM POWDER S.A.
       PANGAEA BIOTECH S.L.

<120>  METHODS AND REAGENTS FOR THE DETECTION OF BIOMOLECULES USING
       LUMINESCENCE

<130>  P9126EP00

<160>  2

<170>  PatentIn version 3.5

<210>  1
<211>  8
<212>  PRT
<213>  Artificial

<220>
<223>  Strep-Tag II

<400>  1

Trp Ser His Pro Glu Phe Gly Lys
1               5


<210>  2
<211>  36
<212>  DNA
<213>  Artificial

<220>
<223>  Oliginucleotide

<400>  2
aacaccgcag catgtcaaga tcacacattt tgggcg                                36